(19) Europäisches Patentamt
European Patent Office
Office européen des brevets

(11) **EP 4 600 635 A1**

(12) **EUROPEAN PATENT APPLICATION**

(43) Date of publication:
**13.08.2025 Bulletin 2025/33**

(21) Application number: **25156530.5**

(22) Date of filing: **07.02.2025**

(51) International Patent Classification (IPC):
**G01N 21/25** *(2006.01)* **G01N 21/27** *(2006.01)*
**G01N 21/82** *(2006.01)*

(52) Cooperative Patent Classification (CPC):
**G01N 21/82; G01N 21/253; G01N 21/272;**
G01N 2021/825

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **09.02.2024 JP 2024018460**

(71) Applicant: **SYSMEX CORPORATION**
**Kobe-shi**
**Hyogo 651-0073 (JP)**

(72) Inventors:
• **KITANO, Keisuke**
**Kobe-shi, Hyogo, 651-0073 (JP)**
• **SAKAYORI, Tasuku**
**Kobe-shi, Hyogo, 651-0073 (JP)**

(74) Representative: **Hoffmann Eitle**
**Patent- und Rechtsanwälte PartmbB**
**Arabellastraße 30**
**81925 München (DE)**

(54) **SAMPLE MEASUREMENT APPARATUS AND SAMPLE MEASUREMENT METHOD**

(57) A sample measurement apparatus according to an embodiment is a sample measurement apparatus (1) for measuring a platelet aggregation function of a blood sample. The apparatus includes: a detector (13) configured to measure optical information of platelet-rich plasma and platelet-poor plasma prepared from the blood sample; a controller (210, 213) configured, based on the optical information of the platelet-rich plasma and the platelet-poor plasma, to acquire information regarding the platelet aggregation function of the blood sample; and a display (4) configured to display the information regarding the platelet aggregation function. The controller (210, 213) is configured to obtain, based on measurement result of the platelet-rich plasma by the detector (13), evaluation information regarding adequacy of the platelet-rich plasma as a specimen, and display the evaluation information on the display (4).

FIG.15

EP 4 600 635 A1

**Description**

BACKGROUND

The disclosure may relate to a sample measurement apparatus and a sample measurement method, and more particularly to a sample measurement apparatus and a sample measurement method for measuring a platelet aggregation function of a blood sample.

In a related art, a platelet aggregation function test has been performed using a blood coagulation test apparatus. To measure a platelet aggregation function, two types of centrifugation are performed on a whole blood sample taken from a patient so as to prepare a platelet rich plasma (PRP) sample which contains a large amount of platelets and a platelet poor plasma (PPP) sample which is substantially free of platelets. The platelet aggregation function of the blood sample is calculated based on optical information obtained by measuring the platelet poor plasma sample and optical information obtained by measuring the platelet rich plasma sample to which a reagent that induces platelet aggregation has been added. However, when the platelet count in the platelet-rich plasma sample is low (less than $150 \times 10^9$/L), there may be a risk that correct results of the platelet aggregation function may not be obtained.

(Non-Patent Document 1)

International Society on Thrombosis and Haemostasis (ISTH) guidelines, "Recommendations for the standardization of light transmission aggregometry: a consensus of the working party from the platelet physiology subcommittee of SSC/ISTH" Journal of Thrombosis and Haemostasis, 11: 1183-1189, 2013

SUMMARY

In order to accurately measure a platelet aggregation function, it may be desirable to use a platelet-rich plasma sample having a platelet count of $150 \times 10^9$/L or more. For this reason, the platelet count of the prepared platelet-rich plasma sample has been measured using a blood cell counter different from the blood coagulation test apparatus, to confirm whether or not the sample is suitable for testing the platelet aggregation function. This confirmation process may be time-consuming and laborious.

A first aspect of one or more embodiments of the disclosure may be a sample measurement apparatus for measuring a platelet aggregation function of a blood sample. The apparatus (1) may include: a detector (13) configured to measure optical information of platelet-rich plasma and platelet-poor plasma prepared from the blood sample; a controller (210, 213) configured, based on the optical information of the platelet-rich plasma and the platelet-poor plasma, to acquire information regarding the platelet aggregation function of the blood sample; and a display (4) configured to display the information regarding the platelet aggregation function. The controller (210, 213) is configured to obtain, based on measurement result of the platelet-rich plasma by the detector (13), evaluation information regarding adequacy of the platelet-rich plasma as a specimen, and display the evaluation information on the display (4).

A second aspect of one or more embodiments of the disclosure may be a sample measurement method for measuring a platelet aggregation function of a blood sample. The method may include: acquiring optical information of platelet-poor plasma and platelet-rich plasma of the blood sample; acquiring information about the platelet aggregation function of the blood sample, based on the optical information obtained from the platelet-poor plasma and the platelet-rich plasma; and acquiring evaluation information about adequacy of the platelet-rich plasma as a specimen, based on the optical information obtained from the platelet-rich plasma.

According to the sample measurement apparatus and the sample measurement method according to the aspects described above, the specimen adequacy of the platelet-rich plasma can be easily determined.

BRIEF DESCRIPTION OF DRAWINGS

FIG. 1 is a diagram illustrating an appearance of a sample measurement apparatus according to an example of an embodiment.
FIG. 2 is a plan view illustrating a configuration of the sample measurement apparatus.
FIG. 3 is a block diagram illustrating a configuration of the sample measurement apparatus.
FIG. 4 is a diagram illustrating an example of a main menu screen;
FIG. 5 is a diagram illustrating an example of an order screen.
FIG. 6 is a flow chart illustrating an example of a sample measurement method.
FIG. 7 is a flow chart illustrating an example of a sample measurement method.

FIG. 8 is a flow chart illustrating an example of a sample measurement method.

FIG. 9 is a diagram illustrating an example of a setting screen for specimen adequacy evaluation.

FIGS. 10A and 10B are diagrams illustrating relationships between initial absorbances and platelet counts of platelet-rich plasma samples.

FIGS. 11A and 11B are diagrams illustrating initial absorbance distributions of platelet-rich plasma samples.

FIG. 12 is a diagram illustrating an example of a setting screen for specimen adequacy evaluation.

FIG. 13 is a diagram illustrating an example of a setting screen for a specimen adequacy evaluation.

FIG. 14 is a diagram illustrating an example of a setting screen for specimen adequacy evaluation.

FIG. 15 is a flow diagram illustrating an example of a sample measurement method including specimen adequacy evaluation.

FIG. 16 is a diagram illustrating the change in absorbance of a sample over time after the addition of an inducer.

FIG. 17 is a flow diagram illustrating an example of a sample measurement method including specimen adequacy evaluation.

FIG. 18 is a flow diagram illustrating an example of a sample measurement method including specimen adequacy evaluation.

FIG. 19 is a diagram illustrating an example of a measurement result display screen.

FIG. 20 is a diagram illustrating an example of a measurement result display screen.

DETAILED DESCRIPTION

Hereinafter, embodiments of a sample measurement apparatus and a sample measurement method are described in detail with reference to the drawings. In the respective drawings referenced herein, the same constituents are designated by the same reference numerals and duplicate explanation concerning the same constituents is omitted. Embodiments described below are merely examples, and the invention is not limited to the embodiments described below. The invention also includes configurations that are obtained by selectively combining components of multiple embodiments and modifications thereof described below.

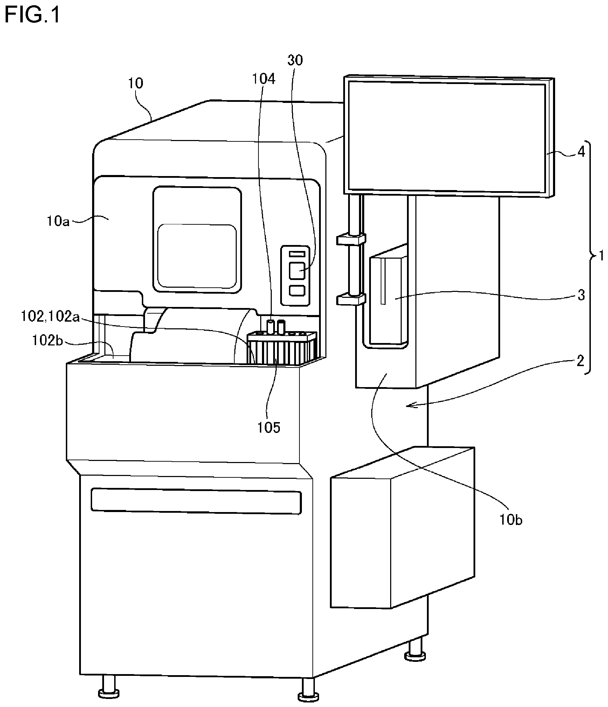

FIG. 1 is a perspective view illustrating an appearance of a sample measurement apparatus 1 according to an example of an embodiment. As illustrated in FIG. 1, the sample measurement apparatus 1 includes a housing 10 having a substantially rectangular parallelepiped outer shape. The sample measurement apparatus 1 is a blood coagulation measurement apparatus configured to measure the platelet aggregation function of a blood sample. The housing 10 includes a front cover 10a that is configured to be opened and closed with respect to the apparatus. The front cover 10a is slidable or rotatable up and down to open the front cover 10a, to thereby expose the inside of the apparatus. The sample measurement apparatus 1 also includes a transport unit 102 configured to transport samples, and a measurement start button 30.

The transport unit 102 is provided at the front of and in a center portion in the height direction of the sample measurement apparatus 1. The transport unit 102 includes a rack setting section 102a (or a rack placement section) where a sample rack 105 is to be placed, and a rack collection section 102b to which the sample rack 105 is transported from inside the housing 10 after the measurement. The sample rack 105 can hold plural sample containers 104 each storing a sample. As will be described in more detail later, when the sample measurement operation begins, the sample rack 105 placed in the rack setting section 102a is transported into the housing 10, and after the sample measurement is completed, the sample rack 105 is transported from inside the housing 10 to the rack collection section 102b.

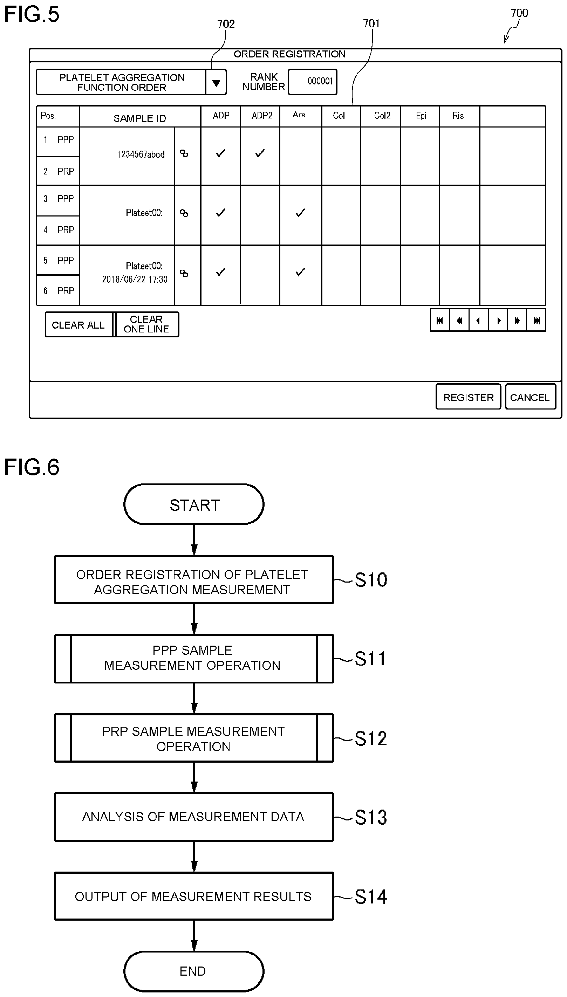

The measurement start button 30 is provided at the front of the sample measurement apparatus 1 and above the rack setting section 102a. In response to the measurement start button 30 being operated by an operator after the sample rack 105 holding sample containers 104 is placed on the rack setting section 102a, the sample rack 105 is transported into the housing 10 and the measurement of the samples is started. In order to manually input sample information such as the sample identification number (sample ID) and a measurement item(s), the sample information is input on an order screen 700 (see FIG. 5 described below) and then the measurement start button 30 is operated (pressed).

The sample measurement apparatus 1 further includes an analyzer 3 (an analysis device 3) and a display 4 (a display unit or a display device). The analyzer 3 is a computer that analyzes the results of the measurements performed in the apparatus main body 2, and is communicably connected to the apparatus main body 2. In an embodiment, the apparatus main body 2 is a unit in which the optical information of a sample is measured, and refers to the parts of the apparatus other than the analyzer 3 and the display 4. In an example illustrated in FIG. 1, the display 4 is attached to the side of the housing 10, and the analyzer 3 is housed in a side rack 10b attached to the rear of the display 4.

The analyzer 3 may be communicably connected to a host computer in which sample information, such as sample IDs, measurement items, and the like, are registered. The display 4 is a display device or a display part that displays information related to platelet aggregation. In addition, the display 4 is a touch panel type display that also functions as an input unit. The display 4 is connected to the analyzer 3 and is communicably connected to the apparatus body 1 via the analyzer 3. For example, operation signals from the display 4, which is the touch panel, are transmitted to the apparatus main body 2 via the analyzer 3. The functions of the analyzer 3 may be built into the apparatus main body 2, and/or the display 4 may be integrated into the apparatus main body 2.

The sample measurement apparatus 1 is an apparatus that analyzes the blood coagulation function of a sample using coagulation method, synthetic substrate method, immunoturbidimetric method, and aggregometry method. The method and configuration of the apparatus for measuring the platelet aggregation function (aggregometry method) will be described in detail below.

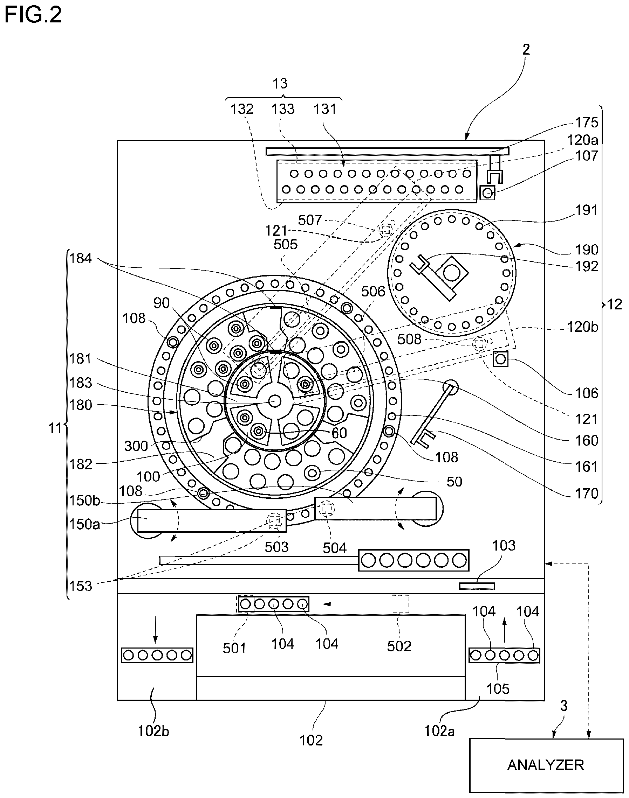

FIG. 2 is a plan view illustrating an internal structure of the sample measurement apparatus 1. As illustrated in FIG. 2, the sample measurement apparatus 1 includes a diluted reagent solution preparation unit 11 that automatically prepares a diluted reagent solution containing a reagent, a measurement specimen preparation unit 12 that prepares a measurement specimen, and a detector 13 that performs optical measurement of the measurement specimen. The diluted reagent solution is prepared by mixing a reagent that induces platelet aggregation in a sample with a diluent such as physiological saline. The measurement specimen is prepared, for example, by mixing the diluted reagent solution prepared by the diluted reagent solution preparation unit 11 with a sample. Samples used in measuring the platelet aggregation function are a platelet-rich plasma (PRP) sample and a platelet-poor plasma (PPP) sample. The detector 13 measures optical information of PRP and PPP samples which are prepared from a blood sample.

The PRP and PPP samples are prepared by performing two different centrifugation procedures on the whole blood sample taken from a patient. The PRP sample is, for example, a supernatant obtained by centrifuging the blood containing an anticoagulant such as sodium citrate at 200×g for 10 minutes. Note that "×g" is the unit of centrifugal force. The PPP sample is, for example, a supernatant obtained by centrifuging blood containing the anticoagulant at 200×g for 10 minutes and then centrifuging it further at 1500×g for 15 minutes. The sample container 104 that contains the PRP sample and the sample container 104 that contains the PPP sample are arranged in a pair in the sample rack 105.

The reagent includes a substance that induces platelet aggregation (an inducer). An example of the inducer includes adenosine diphosphate (ADP), collagen, epinephrine, arachidonic acid, ristocetin, protease-activated receptor 1-activating peptide (PAR1-AP), or the like. For ADP, Revohem (registered trademark) ADP can be used. For collagen, Revohem Collagen can be used. For epinephrine, Revohem Epinephrine can be used. For arachidonic acid, Revohem Arachidonic acid can be used. For ristocetin, Revohem Ristocetin can be used.

The reagent containing the inducer is dispensed only into the container containing the PRP sample. As will be described in more detail below, the measurement specimen preparation unit 12 dispenses the diluted reagent solution containing the reagent prepared by the diluted reagent solution preparation unit 11 into the reaction container 108 containing the PRP sample, and dispenses the diluted solution into the reaction container 108 containing the PPP sample. In addition, the measurement specimen preparation unit 12 heats the reaction container 108 containing the PRP sample to a predetermined temperature before dispensing the diluted reagent solution in order to promote the reaction between the inducer and the platelets in the sample and to make the reaction uniform. Note that the reaction container 108 containing the PPP sample is not heated. Furthermore, an evaluation of a measurement specimen adequacy (specimen adequacy evaluation) is performed on the measurement specimen containing the PRP sample.

The diluted reagent solution preparation unit 11 includes a reagent preparation table 180. The reagent preparation table 180 is a circular table. A plurality of container racks 100, 300 are arranged on the reagent preparation table 180 along the circumferential direction of the table. Arranged in the container racks 100 and 300 are reagent containers 90, which contain a solution with a reagent, diluent containers 50, which contain a diluent, and diluted reagent solution containers 60. The arrangement of the reagent containers 90, the diluent containers 50, and the diluted reagent solution containers 60 in the container racks 100 and 300, and the arrangement of the container racks 100 and 300 on the reagent preparation table 180 are performed by an operator with the front cover 10a being opened to expose the inside of the apparatus.

The reagent preparation table 180 includes a first table 181, which is circular in shape in plan view, and a second table 182, which is annular in shape in plan view and provided on the outer periphery of the first table 181. In an example illustrated in FIG. 1, four container racks 100 are arranged in the circumferential direction on the first table 181. The second table 182 includes three large container racks 300 arranged along the circumferential direction. Each of the first table 181 and the second table 182 is configured to rotate independently in the circumferential direction thereof around a rotation axis 183 by a rotation mechanism equipped with an electric motor, so that the first table 181 and the second table 182 can move, by the rotations thereof, the reagent containers 90, the diluent containers 50, and the diluted reagent solution containers 60 to their respective predetermined positions.

The diluted reagent solution preparation unit 11 includes reagent information readers 184 that read information from the reagent containers 90, the diluent containers 50, the diluted reagent solution containers 60, and the container racks 100 and 300. For example, this information is assigned to each of the containers and the racks via a barcode, and the reagent information readers 184 include barcode readers. The first table 181 and the second table 182 can move the container racks 100 and 300 and the containers held in the racks to reading positions facing the reagent information readers 184. By reading the information with the reagent information readers 184, the positions of the reagent containers 90, the diluent containers 50, and the diluted reagent solution containers 60 on the reagent preparation table 180 can be identified.

The information assigned to the reagent container 90 includes, for example, its identification number (ID), the reagent name, type, concentration, lot number, expiration date, and other relevant details. The information assigned to the diluent container 50 includes, for example, its identification number (ID), the diluent name, type, lot number, expiration date, and other relevant details. The information assigned to the diluted reagent solution container 60 includes, for example, its identification number (ID), and the other relevant details. The information assigned to the container racks 100 and 300 includes, for example, their identification numbers (IDs), and the other relevant details.

The diluted reagent solution preparation unit 11 includes first dispensers 150a and 150b. Each of the first dispensers 150a and 150b includes a dispenser arm that rotatably holds an aspiration tube 153 for dispensing. The aspiration tube 153 is connected to a pump and is configured to aspirate a predetermined amount of fluid and dispense a predetermined amount of fluid. Each of the first dispensers 150a and 150b is configured to move the aspiration tube 153 thereof to a position above a diluent container 50 to aspirate a predetermined amount of diluent from the diluent container 50, and dispense a predetermined amount of diluent into a diluted reagent solution container 60. Further, each of the first dispensers 150a and 150b is configured to move the aspiration tube 153 thereof to a position above a reagent container 90 to aspirate a predetermined amount of reagent from the reagent container 90, and dispense a predetermined amount of reagent into the diluted reagent solution container 60. As a result, the reagent and the diluent are mixed in the diluted reagent solution container 60, and thus a diluted reagent containing the reagent is prepared in the diluted reagent solution container 60.

As described above, the sample measurement apparatus 1 includes the transport unit 102 including the rack setting section 102a and the rack collection section 102b. The transport unit 102 transports the sample rack 105 that is placed by an operator on the rack setting section 102a into the interior of the housing 10 and places the sample containers 104 held in the sample rack 105 at predetermined sample aspiration positions 501 and 502. The transport unit 102 also includes a sample information reader 103 provided to the transport path of the sample rack 105. The sample information reader 103 includes a barcode reader.

A label with sample information, such as a barcode, is attached to each of the sample containers 104. The sample information includes, for example, the sample ID, type, provider, measurement items, and other relevant details. Information regarding the sample type includes information as to whether the sample is a PRP sample or a PPP sample. In a case in which the sample information is registered in the host computer, the operator does not need to input the sample information through the order screen 700, but simply places the sample rack 105 on the rack setting section 102a of the transport unit 102 and operates the measurement start button 30. In this case, the sample information reader 103 reads the barcodes of the sample containers 104 held in the sample rack 105, queries the host computer for information necessary for the measurement, and automatically registers the measurement order.

The measurement specimen preparation unit 12 includes a rotation table 160 (or a turntable) that is configured to transport the reaction containers 108. The rotation table 160 is disposed on the outer periphery of the reagent preparation table 180. The rotation table 160 is in a ring shape in plan view and is configured to rotate in a circumferential direction thereof. The rotation table 160 includes holding holes 161 arrayed along the circumferential direction. One reaction container 108 can be placed in each of the holding holes 161. The PRP sample and the diluted reagent solution prepared by the diluted reagent solution preparation unit 11 are dispensed into the reaction container 108, so as to prepare a measurement specimen in the reaction container 108. The reaction container 108 is a cuvette, for example. A stirrer bar is placed in the reaction container 108 beforehand.

A sample is dispensed into the reaction container 108 by the first dispenser 150a, 150b of the diluted reagent solution preparation unit 11. The first dispenser 150a is configured to move the aspiration tube 153 thereof to aspirate a predetermined amount of a sample from the sample container 104 that is placed at the sample aspirating position 501 of the transport unit 102. The first dispenser 150b is configured to move the aspiration tube 153 thereof to aspirate a predetermined amount of a sample from the sample container 104 that is placed at the sample aspirating position 502 of the transport unit 102. The first dispenser 150a, 150b is configured, after having aspirated the sample, to dispense the sample into the reaction container 108 that is arranged at the sample dispensing position 503, 504 on the rotation table 160.

The measurement specimen preparation unit 12 includes a gripping mechanism 170 configured to transport the reaction containers 108, and a heating table 190 that is configured to hold and heat the reaction containers 108. The gripping mechanism 170 is configured to grip and transport the reaction container 108, remove the reaction container 108 containing the PRP sample from the holding hole 161 of the rotation table 160, and place the reaction container to the heating table 190. Further, the gripping mechanism 170 is configured to place the reaction container 108 containing the PPP sample at the reagent dispensing position 508.

The heating table 190 is a circular table with a built-in heater, and includes a plurality of holding holes 191 for holding a plurality of reaction containers 108 each containing the PRP sample, and a gripping mechanism 192 for gripping and transporting the reaction containers 108. The plurality of holding holes 191 are arranged along the circumferential direction of the heating table 190. The heating table 190 is rotatable in a circumferential direction. The heating table 190 is configured to rotate to transport the reaction containers 108 arranged in the holding holes 191 in the circumferential direction thereof while heating the reaction containers 108 to a predetermined temperature by the heater of the heating table 190. The gripping mechanism 192 is configured to take out the reaction container 108 from the holding hole 191 and place the reaction container 108 at one of the reagent dispensing positions 507 and 508.

The measurement specimen preparation unit 12 includes second dispensers 120a and 120b. Each of the second dispensers 120a and 120b includes an aspiration tube 121 for dispensing. Each of the second dispensers 120a and 120b is configured to move the aspiration tube 121 thereof to a position above the diluted reagent solution container 60 that is placed at a predetermined reagent aspiration position 505, 506 on the reagent preparation table 180, and aspirate a predetermined amount of the diluted reagent from the diluted reagent solution container 60. Thereafter, the second dispenser 120a, 120b is configured to move to a position above the reaction container 108 that is arranged at the reagent dispensing position 507, 508, and dispense a predetermined amount of the diluted reagent solution into the reaction container 108 containing the PRP sample. As a result, the diluted reagent solution and the PRP sample are mixed in the reaction container 108, and thus a measurement specimen containing a predetermined concentration of the reagent is prepared. Note that a diluent is dispensed at the reagent dispensing position 508 into the reaction container 108 containing the PPP sample.

The measurement specimen preparation unit 12 further includes a gripping mechanism 175 configured to transport the reaction container 108. The gripping mechanism 175 includes a transfer mechanism configured to grip the reaction container 108 and transfer the reaction container 108 in X, Y, and Z directions being the three axis directions orthogonal to each other. The gripping mechanism 175 is configured to transfer the reaction container 108 containing the measurement specimen from the reagent dispensing position 507, 508 to a container placement part 131 of the detector 13. The gripping mechanism 175 is further configured to transfer the reaction container 108 after the measurement from the container placement part 131 to a waste outlet 106.

The detector 13 is configured to measure the absorbance or transmittance of the measurement specimens. The detector 13 includes the container placement part 131 to which the reaction containers 108 containing the measurement specimens are to be placed, a light transmitter 132 to irradiate the reaction container 108 with light for signal detection, and a light receiver 133 arranged opposite the light transmitter 132 across the reaction containers 108. The container placement part 131 includes a stirring mechanism that rotates the stirrer bar in the reaction container 108. The detector 13 is provided with a plurality of container placement parts 131. In this case, the measurement specimens in the multiple reaction containers 108 can be measured simultaneously.

The detector 13 measures the change in absorbance or transmittance over time during the process of aggregation reaction of platelets and the like in the measurement specimen in the reaction container 108 arranged in the container placement part 131. The light transmitter 132 emits light to the measurement specimen in the reaction container 108 placed in the container placement part 131. The light transmitter 132 includes a light source such as a light emitting diode or a halogen lamp. The light receiver 133 receives transmitted light or scattered light that is irradiated onto the measurement specimen in the reaction container 108, and outputs an electrical signal according to the amount of the received light. The light receiver 133 includes a photoelectric conversion element that converts the received light into an electrical signal, and transmits the electrical signal to the analyzer 3.

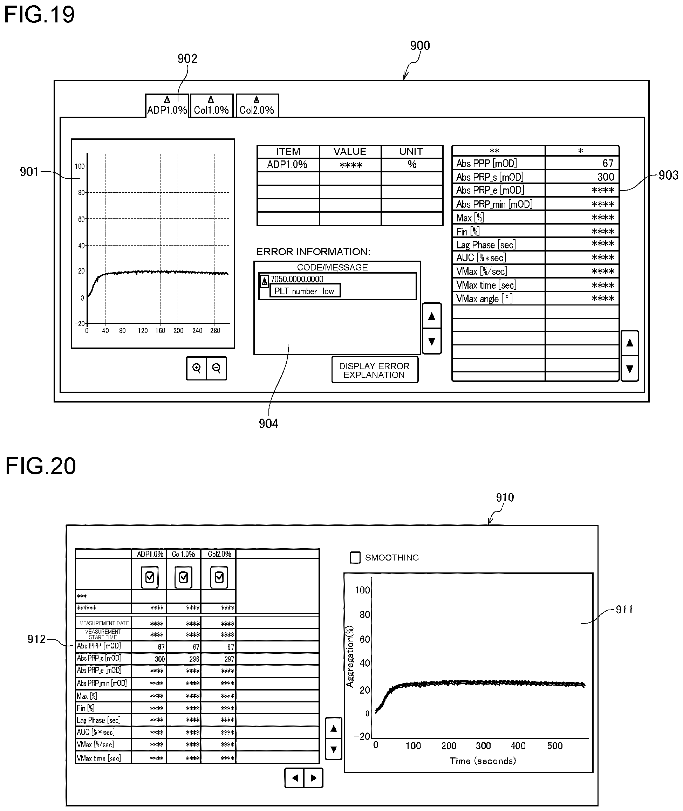

The analyzer 3 analyzes the platelet aggregation function of the sample based on the electrical signal output from the light receiver 133, that is, based on the measurement results of the optical information of the sample acquired by the detector 13. For example, the analyzer 3 generates a reaction curve such as being illustrated in FIG. 19 described below, based on the absorbance or the transmittance of the measurement specimen, and calculates vWF:RCo (von Willebrand factor Ristocetin Cofactor) activity, adenosine diphosphate (ADP) maximum aggregation rate, collagen maximum aggregation rate, epinephrine maximum aggregation rate, arachidonic acid maximum aggregation rate, ristocetin maximum aggregation rate, and protease-activated receptor 1-activating peptide (PAR1-AP) maximum aggregation rate, and etc. The analyzer 3 also performs the specimen adequacy evaluation based on the measurement results of the PRP sample.

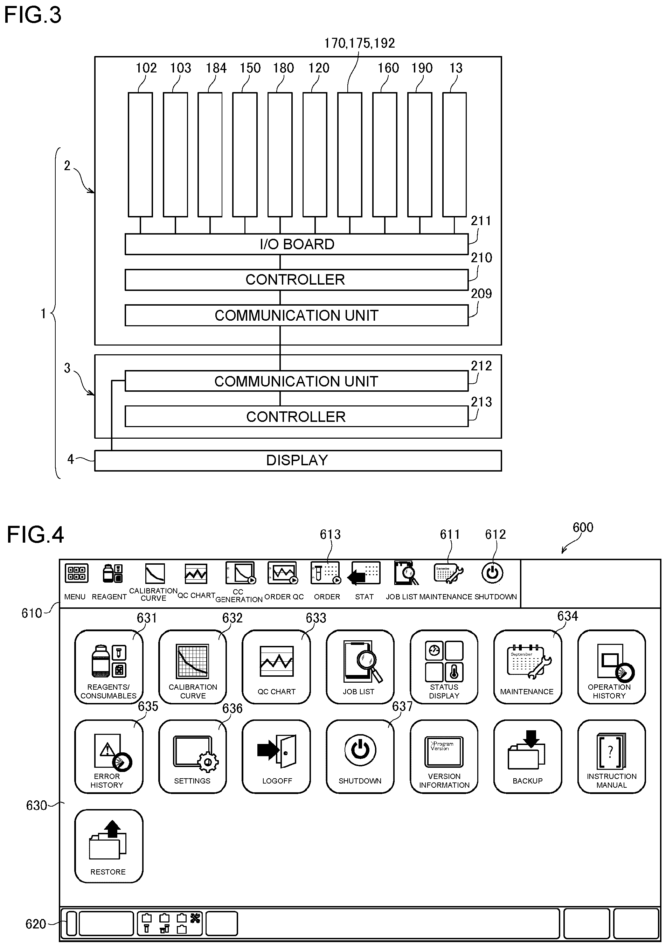

FIG. 3 is a block diagram illustrating configurations of the apparatus main body 2 and the analyzer 3. As illustrated in FIG. 3, the apparatus main body 2 includes a communication unit 209 (or a communication interface), and the analyzer 3 includes a communication unit 212 (or a communication interface). The apparatus main body 2 and the analyzer 3 transmit and receive information via the communication units 209 and 212. A controller 210 includes a processor such as a central processing unit (CPU) or a field-programmable gate array (FPGA), and a storage device such as a read only memory (ROM), a random access memory (RAM), and a hard disk.

The processor of the controller 210 executes a control program stored in the storage device to control each component of the apparatus main body 2 via an I/O board 211. The controller 210 controls, for example, the dispensing operation of the second dispenser 120, the dispensing operation of the first dispensers 150a, 150b, the operation of the reagent preparation table 180, the operation of each table and the operation of each gripping mechanism, the operation of the detector 13, and the like.

The analyzer 3 includes a controller 213 including a processor such as a CPU, and storage devices such as a ROM, a RAM, and a hard disk. In an embodiment, the controller 213 of the analyzer 3 analyzes the platelet aggregation function of the sample based on the electrical signal output from the light receiver 133 of the apparatus main body 2, and also analyzes the specimen adequacy of the measurement specimen containing the PRP sample. The display 4 is connected to the analyzer 3. The analysis results by the analyzer 3 are displayed on the display 4, such as the measurement result of the platelet aggregation function and the evaluation result of the specimen adequacy such as being illustrated in FIGS. 19 and 20 described below.

In an example illustrated in FIG. 3, a configuration is described in which two controllers 210 and 213 are provided, but the number of controllers is not particularly limited. For example, a single controller may control both the apparatus main body 2 and the analyzer 3, or three or more controllers may control the apparatus main body 2 and the analyzer 3. The locations of the controllers are also not particularly limited, and may be located in only one of the apparatus main body 2 and the analyzer 3. As described above, the functions of the analyzer 3 may be built into the apparatus main body 2.

FIG. 4 is a diagram illustrating a main menu screen 600. FIG. 5 is a diagram illustrating an order screen 700 for platelet aggregation function measurement. The main menu screen 600 and the order screen 700 are displayed on the display 4.

As illustrated in FIG. 4, the main menu screen 600 includes: a toolbar 610 including buttons for major functions; a status display area 620 including indicators indicating information such as the status of the apparatus, the status of the host computer, and the remaining amounts of the reagents, the consumables, etc.; and a menu icon display area 630. The toolbar 610 includes a maintenance button 611, which is operated when performing maintenance and inspection of the apparatus; a shutdown button 612, which is operated to shut down the apparatus, an order button 613 to display an order screen 700, and the like.

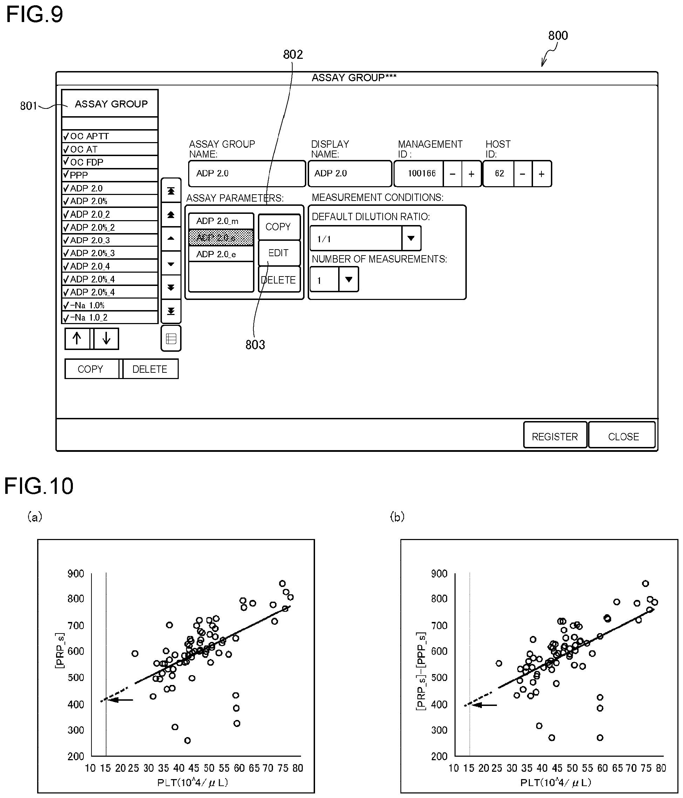

In the menu icon display area 630, a plurality of buttons (icons) for performing various operations and displaying information may be displayed, and the same buttons as those included in the toolbar 610 may be displayed. The menu icon display area 630 includes a reagent consumables button 631, a calibration curve button 632, a QC chart button 633, a maintenance button 634, an error history button 635, a setting button 636, a shutdown button 637, and the like. The setting button 636 is a button to display a setting screen for the adequacy evaluation of the measurement specimen (see FIG. 9 described below). The setting button 636 is operated when performing the specimen adequacy evaluation.

As illustrated in FIG. 5, the order screen 700 includes an order registration section 701 for registering sample information such as a sample ID and measurement items, and a measurement type selection section 702 for displaying the currently selected measurement type. When the sample information is not registered in the host computer, the operator needs to input the sample information required for the measurement into the order registration section 701. Further, the operator selects the measurement type through the measurement type selection section 702. The measurement type selection section 702 is provided with pull-down buttons, which function as buttons for selecting one of the measurement types. In an example illustrated in FIG. 5, the words "Platelet Aggregation Order" are displayed in the measurement type selection section 702, indicating that platelet aggregation function measurement has been selected as the measurement type.

The order registration section 701 is displayed in a table format in which sample information can be input. In measuring the platelet aggregation function, a PRP sample and a PPP sample are placed in the sample rack 105 in a pair, and the paired PRP and PPP samples are managed with the same sample ID. In the order registration section 701, a plurality of measurement items are displayed. The operator can select desired measurement items for each sample from the table in the order registration section 701, and the selected measurement items are registered as an order. Even when specimen adequacy evaluation is performed prior to platelet aggregation function measurement, such an order registration is also performed based on the information read by the sample information reader 103 or based on the information entered on the order screen 700.

Hereinafter, a method of measuring a platelet aggregation function is described with reference to FIGS. 6 through 8.

As illustrated in FIG. 6, the method for measuring the platelet aggregation function includes steps of registering a measurement order (Step S10), preparing measurement specimens and measuring optical information of the measurement specimens (Steps S11 and S12), and analyzing and outputting the measurement results (Steps S13 and S14). In Step S10, the controller 210 controls the sample information reader 103 to read barcodes on sample containers 104, and queries the host computer for information required for the measurement so as to register an order. When the sample information has not been registered in the host computer, the controller 210 performs the order registration based on the information entered on the order screen 700.

In Step S11, the controller 210 controls the diluted reagent solution preparation unit 11 and the measurement specimen preparation unit 12 to prepare a measurement specimen containing a PPP sample, and controls the detector 13 to measure optical information of the measurement specimen containing the PPP sample. In Step S12, the controller 210 controls the diluted reagent solution preparation unit 11 and the measurement specimen preparation unit 12 to prepare a measurement specimen containing a PRP sample, and controls the detector 13 to measure optical information of the measurement specimen containing the PRP sample. Details of Steps S11 and S12 will be described later, but in an embodiment, the absorbance is measured as the optical information of the measurement specimens.

In Step S13, the controller 210 transmits the measurement data acquired in Steps S11 and S12 to the controller 213 of the analyzer 3, and the controller 213 executes an analysis of the measurement data. For example, for the PRP sample, the controller 213 calculates the change in the aggregation rate of the PRP sample over time by setting the initial absorbance immediately after adding the diluted reagent solution to the PRP sample as the aggregation rate of 0% and the absorbance of the PPP sample as the aggregation rate of 100%, and generates a reaction curve (an aggregation waveform in which the absorbance is converted into the aggregation rate) such as being illustrated in FIGS. 19 and 20 described below. Further, the controller 213 calculates the maximum aggregation rate from the maximum change in the absorbance associated with the platelet aggregation reaction.

In Step S14, the controller 213 of the analyzer 3 causes the display 4 to display the measurement results of the platelet aggregation function, such as the reaction curve and the maximum aggregation rate. As will be described in more detail below, a measurement result display screen may display the evaluation results of the specimen adequacy of the PRP sample together with the platelet aggregation function measurement results.

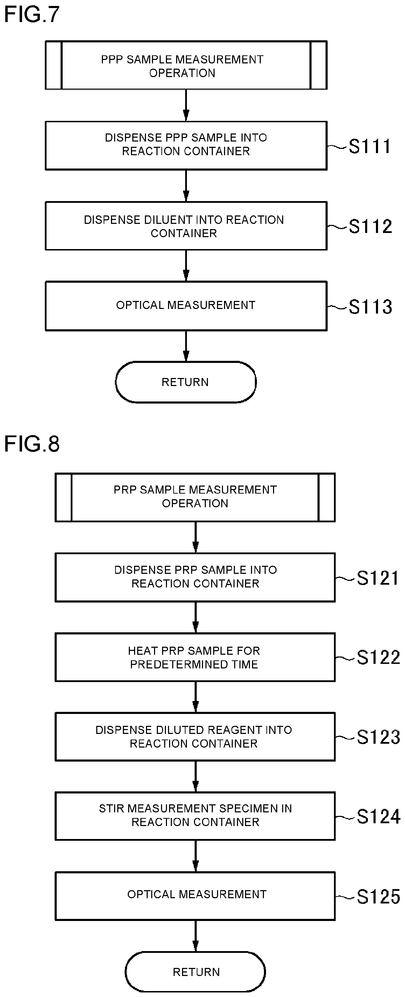

FIG. 7 is a flow chart illustrating an example of the method for measuring the PPP sample in Step S11 of FIG. 6. As illustrated in FIG. 7, in Step S111, the controller 210 controls the first dispenser 150a of the diluted reagent solution preparation unit 11 to aspirate, from a sample container 104 containing a PPP sample, a predetermined amount of the PPP sample, and dispense it into a reaction container 108. In Step S112, the controller 210 controls the second dispenser 120a of the measurement specimen preparation unit 12 to aspirate, from a diluent container 50, a predetermined amount of a diluent and dispense it into the reaction container 108, thereby preparing a measurement specimen containing the PPP sample.

In Step S113, the controller 210 controls the gripping mechanism 175 of the measurement specimen preparation unit 12 to transfer the reaction container 108 containing the diluted PPP sample to the detector 13, and controls the detector 13 to measure the absorbance of the PPP sample for a predetermined period of time.

FIG. 8 is a flow chart illustrating an example of the method for measuring the PRP sample in Step S12 of FIG. 6. As illustrated in FIG. 8, in Step S121, the controller 210 controls the first dispenser 150a to aspirate, from a sample container 104 containing a PRP sample, a predetermined amount of the PRP sample, and dispense it into a reaction container 108 in which a stirrer bar has already been placed. In Step S122, the controller 210 controls the gripping mechanism 170 of the measurement specimen preparation unit 12 to transfer the reaction container 108 containing the PRP sample onto the heating table 190, and heats the reaction container 108 at a predetermined temperature for a predetermined period of time.

In Step S123, the controller 210 controls the second dispenser 120a to aspirate a predetermined amount of diluted reagent solution from a diluted reagent solution container 60 and dispense it into the reaction container 108 containing the PRP sample, thereby preparing a measurement specimen containing the PRP sample. Note that the controller 210 prepares one or more types of measurement specimens for one PRP sample, by dispensing a predetermined reagent(s) at a predetermined concentration(s) based on the measurement item(s) for which the order has been registered. In Step S124, the controller 210 controls the gripping mechanism 175 to transfer the reaction container 108 containing the measurement specimen to the container placement part 131 of the detector 13, and rotates the stirrer bar in the reaction container 108 to stir the measurement specimen therein.

In Step S125, the controller 210 controls the detector 13 to measure the absorbance of the measurement specimen containing the PRP sample and the reagent of the predetermined concentration for a predetermined period of time while stirring the measurement specimen. Note that when plural measurement items with different reagent types and concentrations are registered as the order for one PRP sample, Steps S121 to S125 are repeated according to the number of measurement items. The preparation and measurement of the plural measurement specimens corresponding to the plural measurement items may be carried out simultaneously in parallel.

The function for evaluating the adequacy of a PRP sample as a measurement specimen is detailed below with reference to FIGS. 9 through 20.

FIG. 9 is a diagram illustrating an example of a setting screen for the specimen adequacy evaluation. As illustrated in FIG. 9, a setting screen 800 includes a list display area 801 in which a plurality of measurement items are displayed, and a parameter registration section 802 for confirming, setting, changing, etc. the conditions of each measurement item. The setting screen 800 is a screen for confirming, setting, changing, etc. the conditions of each measurement item of the platelet aggregation function of a sample, and is also possible to register new measurement items and delete registered measurement items on the setting screen 800. Further, when performing evaluating the adequacy of a measurement specimen containing a PRP sample, the setting screen 800 is used for setting information required for the evaluation.

By operating the setting button 636 on the main menu screen 600, the setting screen 800 is displayed on the display 4. In the list display area 801 in the setting screen 800, a plurality of measurement items are displayed aligned vertically on the screen. As the measurement items for the platelet aggregation function, a plurality of items with generally different types and concentrations of inducers, are registered in the sample measurement apparatus 1. In the setting screen 800, by selecting one of the measurement items (a type and a concentration of an inducer) in the list display area 801, the selected measurement item is displayed in a parameter registration section 802. The parameter registration section 802 is provided with an edit button 803. The operator can set or change the conditions of the selected measurement item by operating the edit button 803.

In the sample measurement apparatus 1, evaluation information regarding the specimen adequacy of the PRP sample is obtained based on the measurement results of the measurement specimen containing the PRP sample by the detector 13, and the evaluation information is displayed on the display 4 serving as a display part or a display device. When the platelet count (the number of platelets) in the PRP sample is low, there may be a risk that accurate results will not be obtained in the platelet aggregation function measurement. For this reason, in a related art, the platelet count of a PRP sample is separately measured using a hemocytometer to confirm whether or not the sample is adequate for measuring the platelet aggregation function. On the other hand, with the sample measurement apparatus 1 according to an embodiment, the specimen adequacy of a PRP sample can be easily determined, and the platelet aggregation function can also be measured following the specimen adequacy determination, greatly improving the usability and the reliability of the measurement results.

In an embodiment, the optical information of the PRP sample required to obtain the evaluation information of the specimen adequacy is absorbance or transmittance. Since the sample measurement apparatus 1 is equipped with the detector 13 that measures the absorbance or transmittance of the specimen containing the PRP sample, the absorbance or transmittance obtained by the detector 13 can be used as the evaluation information for the specimen adequacy. In an embodiment, the absorbance of the specimen containing the PRP sample is measured by the function of the controller 210 of the apparatus main body 2. Then, the measurement results are analyzed by the function of the controller 213 of the analyzer 3, and the evaluation information regarding the measurement specimen adequacy is obtained.

In an example illustrated in FIG. 9, in the parameter registration section 802, "ADP2.0_m", "ADP2.0_s", and "ADP2.0_e" are displayed and "ADP2.0_s" is selected among them. When the edit button 803 is operated in the state where "ADP2.0_s" is selected, the conditions of "ADP2.0_s" can be set or changed. In "ADP2.0_s", "ADP" represents the type of inducer, "2.0" indicates the concentration of the inducer, and "s" indicates the initial absorbance. The initial absorbance is the absorbance immediately after the addition of the inducer to the PRP sample (for example, within 10 seconds, as described in detail below), and refers to the absorbance in the initial state before the platelet aggregation is detected.

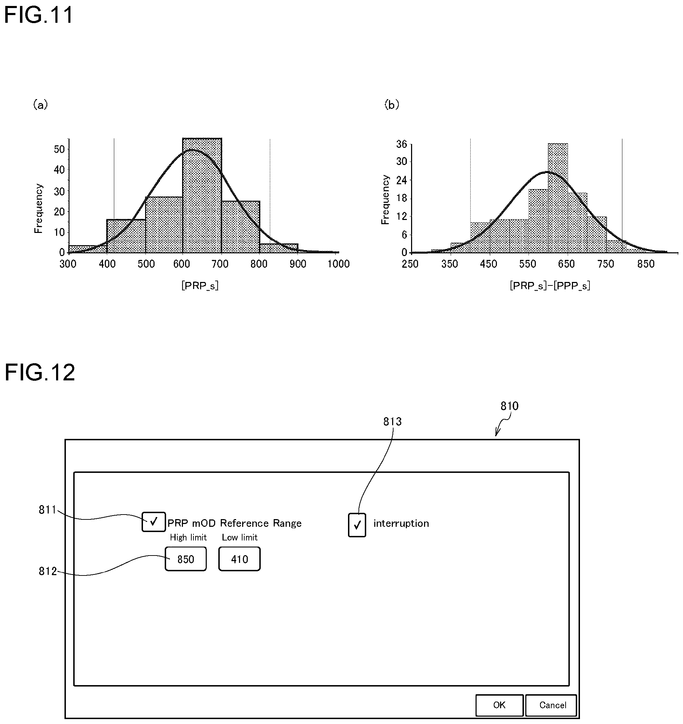

Although described in detail below, the adequacy evaluation of the measurement specimen containing the PRP sample is performed, for example, using the initial absorbance of the measurement specimen containing the PRP sample. Therefore, when performing the specimen adequacy evaluation, the conditions of the initial absorbance are set on the setting screen 800. Since the initial absorbance does not vary significantly depending on measurement items (types and concentrations of inducers), the conditions of the initial absorbance may be set for each measurement item, or may be set to be common to all measurement items. When the edit button 803 is operated in the state where "ADP2.0_s" is selected, for example, a setting screen 810 (see FIG. 12 described later) for setting an evaluation reference value of the initial absorbance is displayed.

FIGS. 10A and 10B are diagrams illustrating relationships between the initial absorbances ([PRP_s]) of measurement specimens containing PRP samples and their platelet counts (PLT). FIGS. 11A and 11B are diagrams illustrating the initial absorbance distributions of measurement specimens containing PRP samples. As will be described in more detail later, it can be understood from the data illustrated in FIGS. 10A, 10B, 11A and 11B that the absorbance (initial absorbance) of a specimen containing a PRP sample obtained by the detector 13 can be used as evaluation information for specimen adequacy. The initial absorbance of a specimen containing a PRP sample is the absorbance obtained by the detector 13, and is the absorbance in the initial state after the addition of an inducer to the PRP sample and before the platelet aggregation is detected by the detector 13, as described above.

The initial absorbance is measured at a wavelength of 660 nm using a blood coagulation measuring device (a full-automated blood coagulation analyzer CS-5100 manufactured by Sysmex Corporation). The platelet count is measured using a blood cell counter (a multi-item automated blood analyzer XS-1000i manufactured by Sysmex Corporation). FIGS. 10A and 10B show the recommended lower limit ($15 \times 10^4/\mu L$) of the platelet count contained in a PRP sample according to the International Society on Thrombosis and Haemostasis (ISTH) guidelines.

The PRP and PPP samples used to generate the data illustrated in FIGS. 10A and 10B are prepared from the blood of 70 healthy volunteers. The method for preparing the PRP and PPP samples of each healthy volunteer is as follows.

(1) Blood is collected from a healthy volunteer using 3.2% sodium citrate blood collection tubes (Venoject RRtubes (registered trademark) manufactured by Terumo Corporation).
(2) The blood of two blood collection tubes is dispensed into a 10 mL centrifuge tube (sterile round-bottomed Spitzer, Eiken Chemical Co., Ltd.) and centrifuged at $200 \times g$ for 10 minutes using a high-speed refrigerated centrifuge (Model 7000, Kubota Shoji Co., Ltd.). A portion of the supernatant of the centrifuged blood is collected and used as a PRP sample.

(3) The remaining supernatant is centrifuged at 1,500×g for an additional 15 minutes, and the supernatant of the further centrifugation is collected and used as a PPP sample.

FIG. 10A illustrates the relationship between the platelet count (PLT) and the initial absorbance [PRP_s] of the specimen containing the PRP sample, and FIG. 10B illustrates the relationship between the platelet count (PLT) and the value of [PRP_s] - [PPP_s] obtained by subtracting the initial absorbance [PPP_s] of the specimen containing the PPP sample from the initial absorbance [PRP_s] of the specimen containing the PRP sample. Since each of the PRP and PPP samples contains components other than platelets, such as lipids, proteins, and amino acids, the effects of these components can be eliminated by calculating the value of [PRP_s] - [PPP_s]. As illustrated in FIGS. 10A and 10B, a positive correlation is obtained between the initial absorbance and the platelet count in all cases, suggesting that the adequacy of the PRP specimen for measuring the platelet aggregation function can be determined based on the initial absorbance value of the PRP specimen without measuring the platelet count of the PRP specimen. Accordingly, by setting a reference value and comparing the initial absorbance of the PRP specimen with the reference value, it is possible to determine whether or not the specimen is adequate for the measurement.

The regression equation for determining the platelet count from the initial absorbance is as follows: Although details will be described later, either of the following regression equation 1 or 2 can be used to evaluate specimen adequacy.

$$\text{Equation 1: PLT} = [PRP\_s]/5.71 - 336.92/5.71$$

$$\text{Equation 2: PLT} = ([PRP\_s]-[PPP\_s])/5.70 - 317.48/5.70$$

FIG. 11A illustrates the distribution of [PRP_s], and FIG. 11B illustrates the distribution of [PRP_s]-[PPP_s]. The PRP and PPP samples used to generate the data illustrated in FIGS. 11A and 11B are prepared from the blood of 130 healthy volunteers. The preparation method for the PRP and PPP samples is as described above. FIGS. 11A and 11B illustrate the upper and lower limits of the 95% confidence interval.

The 95% confidence interval of the distribution of [PRP_s] illustrated in FIG. 11A is 419 to 828 mOD (Optical Density), and the 95% confidence interval of the distribution of [PRP_s] - [PPP_s] illustrated in FIG. 11B is 401 to 787 mOD. The platelet count calculated by substituting the lower limit of the 95% confidence interval in the distribution of initial absorbance into the regression equation is approximately consistent with the recommended lower limit of the platelet count in the ISTH guidelines. Therefore, it can be understood that the initial absorbance of the specimen acquired by the detector 13 can be used as the evaluation information for the specimen adequacy.

The controller 213 of the analyzer 3 compares the initial absorbance of the PRP sample measured by the detector 13 with the reference value to obtain information for evaluating the specimen adequacy of the PRP sample. The reference value is set, for example, in the range of 400 to 440 mOD. The value set in the range of 400 to 440 mOD is the lower limit reference value (lower limit), and as described above, corresponds to the recommended lower limit of the platelet count in the ISTH guidelines. Therefore, by using this lower limit, it is possible to perform the specimen evaluation based on the recommended lower limit in the ISTH guidelines. The value of [PRP_s] - [PPP_s] may be used to evaluate the specimen adequacy. In this case, the lower limit is set, for example, in the range of 380 to 420 mOD.

The controller 213 outputs error information when the initial absorbance of the PRP sample is below the reference value. The error information is evaluation information that indicates that the PRP sample used is not adequate (suitable) as a specimen. The error information will be described in detail later. An upper limit reference value (upper limit) may further be set for the specimen adequacy evaluation. Based on the above-mentioned study results, the upper limit is set, for example, in the range of 800 to 840 mOD. The value of [PRP_s] - [PPP_s] may be used to evaluate the specimen adequacy. In this case, the upper limit is set in the range of 760 to 800 mOD, for example.

The controller 213 may compare the platelet count with a reference value to obtain evaluation information regarding the specimen adequacy of the PRP sample. When the platelet count of the PRP sample is below the reference value, the controller 213 outputs error information. The platelet count of the PRP sample is calculated based on the initial absorbance of the PRP sample measured by detector 13, specifically using the regression equation 1 described above. In this case, the lower limit recommended by the ISTH guidelines can be used as the lower limit. In addition, an upper limit for the platelet count may be further set based on the regression equation 2 described above.

FIG. 12 is a diagram illustrating the setting screen 810, which is an example of a screen for setting the reference value for the specimen adequacy evaluation. As illustrated in FIG. 12, the setting screen 810 includes a selection button 811 for selecting whether or not to evaluate the specimen adequacy based on the initial absorbance of the PRP sample, and a reference value input section 812 for inputting the reference values required for the evaluation. The selection button 811 includes a check box. When the operator checks the checkbox of the selection button 811, the evaluation of the specimen adequacy based on the initial absorbance is enabled (activated). That is, the evaluation of the specimen adequacy is performed. Further, in the state where the check box of the selection button 811 is checked, it is possible to input the reference values into the reference value input section 812.

The reference value input section 812 includes two input sections into which the upper and lower limit reference values can be input. In the setting screen 810, the evaluation conditions based on the initial absorbance of the PRP sample are set, and therefore the reference values of the initial absorbance are input in the reference value input section 812. In an example illustrated in FIG. 12, initial absorbance values of "410" as the lower limit and "850" as the upper limit are input. The reference values may be arbitrary values input by the operator, or may be values selectable from among predetermined values. Also, only the selection button 811 may be displayed on the setting screen, and the reference values may be fixed values registered in advance.

The setting screen 810 further includes a selection button 813 for selecting whether or not to inquire about continuing the measurement based on the evaluation information of the specimen adequacy. The selection button 813 includes a check box. When the operator checks the check box of the selection button 813, an inquiry is made as to whether or not to continue the subsequent measurement based on the evaluation information of the specimen adequacy. The information inquiring whether or not to continue the measurement is displayed on the display 4 together with a button for selecting whether or not to continue the measurement.

When the evaluation result of the specimen adequacy is positive, i.e., when the initial absorbance of the PRP sample is within the range between the upper and lower limit reference values entered in the reference value input section 812, the controller 210 of the apparatus main body 2 continues measuring the platelet aggregation function without executing the inquiry even when the check box of the selection button 813 is checked. From the viewpoint of improving usability, it may be preferable to automatically continue the measurement when the evaluation result of the specimen adequacy is within the reference value range. When the check box of the selection button 813 is not checked, the measurement of platelet aggregation function is continued until the end of the measurement regardless of the evaluation result.

When the evaluation information is acquired that indicates that the PRP sample is not adequate as a specimen , the controller 210 causes the display 4 to display information for confirming the intention to continue the measurement. In this case, the operator can decide whether or not to continue the measurement based on the information. That is, when the evaluation result of the specimen adequacy is negative, an inquiry is made as to whether or not to continue the measurement. As a specific example, when the initial absorbance of the PRP sample falls below the lower limit input in the reference value input section 812, this information is displayed on the display 4 together with a question as to whether or not to continue the measurement.

Based on the measurement result of the PRP sample or the evaluation information of the specimen adequacy, the controller 210 may determine whether or not to prepare a measurement specimen by adding the reagent containing the inducer (the inducer-containing reagent) to the PRP sample, or may determine whether or not to continue measuring the absorbance of the measurement specimen to which the inducer-containing reagent has been added. In a case where the former judgment is to be performed, the specimen adequacy evaluation is performed and then the inducer-containing reagent is added to the PRP sample to prepare a measurement specimen. On the other hand, in a case where the latter judgment is to be performed, the measurement of the optical information of the PRP sample to obtain the evaluation information is performed after preparing the measurement specimen, i.e., within a predetermined period of time after adding the inducer-containing reagent to the PRP sample.

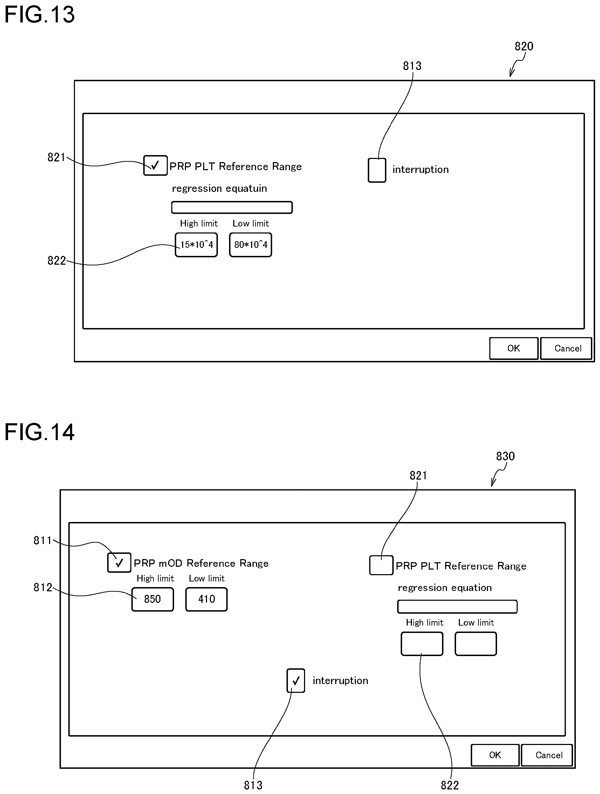

FIG. 13 is a diagram illustrating a setting screen 820 which is another example of a screen for setting reference values for the specimen adequacy evaluation. As illustrated in FIG. 13, the setting screen 820 includes a selection button 821 for selecting whether or not to evaluate the specimen adequacy based on the platelet count of the PRP sample, and a reference value input section 822 for inputting the reference values required for the evaluation. The selection button 821 includes a check box. When the operator checks the check box of the selection button 821, the evaluation of the specimen adequacy based on the platelet count is enabled (activated). Further, in the state where the check box of the selection button 821 is checked, it is possible to input the reference values into the reference value input section 822.

Similar to the setting screen 810, the reference value input section 822 includes two input sections into which upper and lower limit reference values can be input. In the setting screen 820, the evaluation conditions based on the platelet count of the PRP sample are set, and therefore the reference values of the platelet count are input in the reference value input section 822. In an example illustrated in FIG. 13, the platelet count of "$15\times10^4/\mu L$" is input as the lower limit and "$80\times10^4/\mu L$" is input as the upper limit. The reference values may be arbitrary values input by the operator, or may be values selectable from among predetermined values. Also, only the selection button 821 may be displayed on the setting screen, and the reference values may be fixed values registered in advance.

The reference value input section 822 may further display the regression equation for calculating the platelet count from the initial absorbance of the PRP sample. The regression equation may be selectable by the operator from among equations registered in advance. Further, similar to the setting screen 810, the setting screen 820 may include a selection button 813 for selecting whether or not to inquire about continuing the measurement based on the evaluation information of the specimen adequacy. When the evaluation conditions are set based on the setting screen 820, the controller 213 estimates the platelet count from the initial absorbance of the PRP sample, and obtains evaluation information based on the platelet count. Specifically, the platelet count is calculated from the initial absorbance of the PRP sample using the regression equation 1 or 2 described above, and the calculated platelet count is compared with the reference values entered in the reference value input section 822 so as to evaluate the specimen adequacy.

FIG. 14 is a diagram illustrating a setting screen 830 which is another example of a screen for setting reference values for the specimen adequacy evaluation. As illustrated in FIG. 14, the setting screen 830 includes both a selection button 811 for selecting whether or not to evaluate the specimen adequacy based on the initial absorbance of the PRP sample, and a selection button 821 for selecting whether or not to evaluate the specimen adequacy based on the platelet count of the PRP sample. In this case, the operator can select whether to perform the evaluation using the initial absorbance or the platelet count. The setting screen 830 also displays the reference value input section 812 in which the reference values of the initial absorbance are input, the reference value input section 822 in which the reference values of the platelet count are input, and the selection button 813 for selecting whether or not to inquire about continuing the measurement.

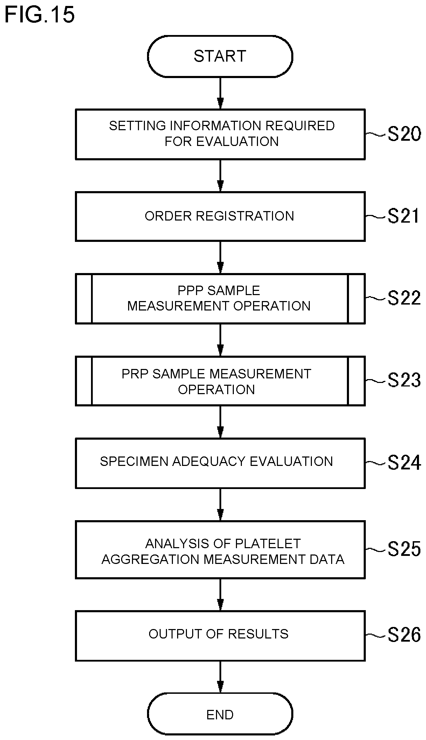

FIG. 15 is a flow diagram illustrating an example of a sample measurement method including specimen adequacy evaluation. Although not illustrated in FIG. 15, the sample measurement method including the specimen adequacy evaluation includes a step of preparing a PPP sample and a PRP sample from a blood sample.

In an example illustrated in FIG. 15, the PPP sample and the PRP sample are measured in the same manner as in a normal platelet aggregation function measurement, and the specimen adequacy of the PRP sample is evaluated. In this case, the measurement operation of the PPP sample (step S22) is the same as or similar to the method illustrated in FIG. 7. The measurement operation of the PRP sample (Step S23) is the same as or similar to the method illustrated in FIG. 8. When the PRP sample measurement in Step S23 is completed, the display 4 displays the measurement result of the platelet aggregation function as well as the evaluation result of the specimen adequacy.

As illustrated in FIG. 15, in order to perform the adequacy evaluation of the measurement specimen containing the PRP sample, the controller 210 sets information required for the evaluation (Step S20). The conditions for the adequacy evaluation are set based on operation of the operator. For example, when the check box of the selection button 811 on the setting screen 810 illustrated in FIG. 12 is checked, the controllers 210 and 213 execute the specimen adequacy evaluation based on the initial absorbance. In the case where the setting screen 820 illustrated in FIG. 13 is applied, the controllers 210 and 213 execute the specimen adequacy evaluation based on the platelet count when the check box of the selection button 821 on the setting screen 820 is checked.

The controller 210 sets the reference values inputted in the reference value input section 812 of the setting screen 810 as judgment references for the specimen adequacy evaluation. Specifically, the lower and upper limits of the initial absorbance are set as the reference values. In the case of the basis of an input in the reference value input section 822 of the setting screen 820, the lower and upper limits of the platelet count are set as the reference values. Note that the reference values may be values registered in advance, or may be automatically set by a function of the controller 210.

In Step S21, the controller 210 controls the sample information reader 103 to read barcodes of sample containers 104. When the sample information is registered in the host computer, the controller 210 inquires of the host computer about information necessary for the measurement, and automatically registers the sample information. For example, when the measurement start button 30 is operated in the state where the sample rack 105 holding the sample containers 104 is placed on the rack setting section 102a, the sample rack 105 is transported into the housing 10 to start the sample measurement, and the measurement order is registered. Note that Step S21 is the same as or similar to Step S10 illustrated in FIG. 6.

The controller 210 prepares a specimen containing a PPP sample and performs the absorbance measurement of the specimen containing the PPP sample (Step S22: acquiring first optical information), and then prepares a specimen containing a PRP sample and performs the absorbance measurement of the specimen containing the PRP sample (Step S23: acquiring second optical information). The measurement specimens are prepared by dispensing the samples from the sample containers 104 in the sample rack 105 arranged on the rack setting section 102a. When measuring the platelet aggregation function, the sample rack 105 holds the sample container 104 containing the PPP sample and the sample container 104 containing the PRP sample having the same sample ID, i.e., from the same donor.

When the measurement of the PRP sample is completed, the controller 213 obtains the evaluation information regarding the specimen adequacy of the PRP sample based on the absorbance of the PRP sample (Step S24). Specifically, the initial absorbance of the PRP sample measured by the detector 13, or the platelet count calculated from the initial absorbance, is compared with the reference value, so as to evaluate the specimen adequacy of the PRP sample. In an example illustrated in FIG. 15, since the measurement of the PRP sample is completed in Step S23, the controller 213 performs an analysis of the platelet aggregation function regardless of the evaluation result of the specimen adequacy (Step S25). The order of Steps S24 and S25 is not particularly limited, and the order may be reversed.

The controller 213 outputs the evaluation result of the specimen adequacy on the display 4 (Step S26). The evaluation result of the specimen adequacy is output together with the platelet aggregation function measurement result. As will be described in more detail later, the evaluation result of the specimen adequacy and the measurement result of the platelet aggregation function are displayed on the same screen. When the initial absorbance of the PRP sample measured by the detector 13, or the platelet count calculated from the initial absorbance, falls below the lower limit reference value, the controller 213 outputs error information indicating that the PRP sample used is not adequate as a specimen.

In an example illustrated in FIG. 15, as described above, after the platelet aggregation function measurement, the evaluation information on the specimen adequacy is obtained using the measurement result, i.e, the optical information of the PRP sample. In this case, the controller 210 measures the absorbance of the PRP sample to obtain the evaluation information within a predetermined time after the addition of the inducer-containing reagent to the PRP sample. The predetermined time is set to, for example, a time period of 2 seconds or more and 10 seconds or less after the addition of the inducer-containing reagent.

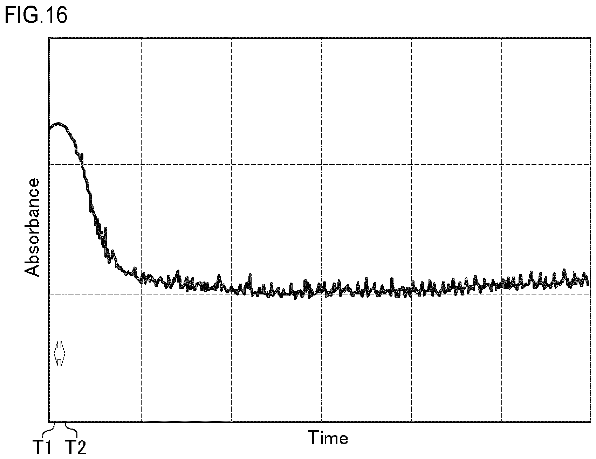

FIG. 16 illustrates the change in absorbance of the PRP sample over time after addition of the inducer-containing reagent. As illustrated in FIG. 16, when the inducer is added to the PRP sample, the platelet aggregation reaction progresses and the absorbance decreases. However, the decrease in absorbance due to the aggregation reaction is detected only after a predetermined time T2 has elapsed since the addition of the inducer. Therefore, by using the initial absorbance within the predetermined time T2 after the addition of the inducer, it is possible to appropriately evaluate the specimen adequacy of the PRP sample to which the inducer has been added. The predetermined time T2 during which the absorbance hardly changes varies depending on the type of inducer, but is, for example, 4 to 10 seconds.

In an embodiment, the inducer-containing reagent is dispensed into the reaction container 108 containing the PRP sample to prepare the measurement specimen in the reaction container 108, and then the reaction container 108 is immediately transferred to the detector 13. Therefore, in order to eliminate the effect of the shock of transport on the measurement, it may be preferable that the measurement of the initial absorbance to obtain evaluation information on the specimen adequacy is performed a predetermined time T1 after the reaction container 108 is transported to the detector 13. An example of the predetermined time T1 is 0.5 to 2 seconds. Therefore, the controller 210 measures the initial absorbance within a period of 2 to 10 seconds after the addition of the inducer-containing reagent to the PRP sample.

The initial absorbance is calculated, for example, by monitoring the absorbance for a predetermined time period from T1 to T2 and linearly approximating the change. By substituting 0 for the time in the linear approximation equation, the initial absorbance can be calculated using this equation. In this case, the influence of variations in the measured values can be suppressed, and the reliability of the evaluation result is further improved. Also, the absorbance at the predetermined time T1 can be defined as the initial absorbance.

The controller 210 may measure the absorbance of the PRP sample for obtaining evaluation information on the specimen adequacy, after preparing the measurement specimen by adding the inducer-containing reagent to the PRP sample and before starting to stir the measurement specimen. The detector 13 is equipped with the stirring mechanism that rotates the stirrer bar in the reaction container 108. The absorbance measurement is performed with the stirrer bar rotating. However, since rotating the stirrer bar promotes the agglutination reaction, the absorbance measurement for evaluating the specimen adequacy may be performed before starting to stir the measurement specimen. For example, after adding the inducer-containing reagent to the PRP sample, the controller 210 measures the initial absorbance of the PRP sample to obtain evaluation information within a predetermined time before the stirring begins.

The controller 213 compares the optical information of the PRP sample with the reference value to obtain evaluation information of the specimen adequacy. The evaluation information may include information that prompts the blood cell counter to measure the platelet count. The evaluation information may also include information regarding the adequacy of the centrifugation conditions used when preparing the PRP sample from the blood sample. The controller 213 may further estimate the possibility of other diseases based on the measurement result or the evaluation information of the PRP sample, and cause the display 4 to display information regarding the other diseases. The information regarding other diseases may be output as part of the specimen adequacy evaluation information.

When the initial absorbance or the platelet count falls below the lower limit reference value or exceeds the upper limit reference value, the controller 213 outputs the error information indicating that the PRP sample used is not adequate as a specimen, as the evaluation information. The error information may include information that prompts the user to measure the platelet count using a blood cell counter, such as text information (message) like "The platelet count may be outside the recommended range for measurement. Please measure the platelet count using a blood cell counter," or an error code indicating the same.

The error information may include information regarding the adequacy of the centrifugation conditions for preparing the PRP sample from the blood sample, such as text information (message) like "The platelet count may be outside the recommended range for measurement. Please check the centrifugation conditions for the sample preparation," or an error code indicating the same. The main cause of the platelet count falling outside the recommended range for measurement may be the centrifugation conditions in the PRP sample preparation. Therefore, by providing the information regarding the centrifugation conditions, it is possible to encourage confirmation of the centrifugation conditions and the sample preparation under appropriate conditions.

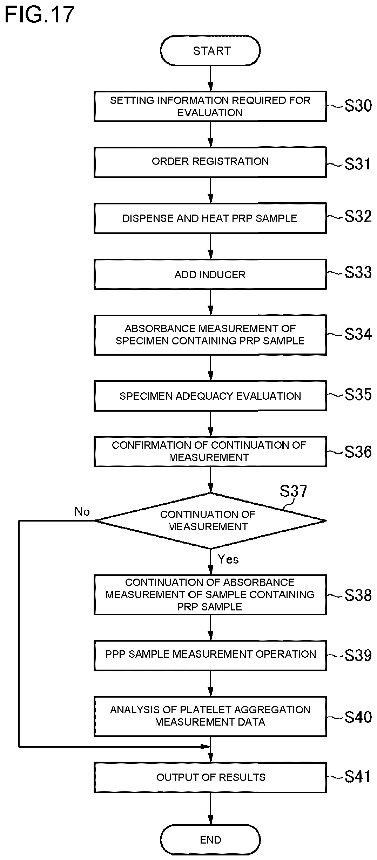

FIG. 17 is a flow diagram illustrating another example of a sample measurement method including specimen adequacy evaluation. An example illustrated in FIG. 17 is common to the example illustrated in FIG. 15 in that after an inducer-containing reagent is added to a PRP sample, the absorbance is measured within a predetermined time to evaluate specimen adequacy. To the contrary, the example illustrated in FIG. 17 differs from the example illustrated in FIG. 15 in that when the evaluation information is obtained indicating that the PRP sample is not adequate as a specimen, information is output to confirm the operator's intention to continue the measurement. Furthermore, in the example illustrated in FIG. 17, the absorbance measurement of the PPP sample is performed after the specimen adequacy evaluation.

As illustrated in FIG. 17, the controller 210 sets information required for specimen adequacy evaluation (Step S30). Step S30 is the same as or similar to Step S20 in FIG. 15. When the measurement start button 30 is operated and a sample rack 105 is thus transported inside the housing 10, the controller 210 controls the sample information reader 103 to read barcodes of sample containers 104 in the sample rack 105 and register a measurement order (Step S31). Note that Step S31 is the same as or similar to Step S10 in FIG. 6 and Step S21 in FIG. 15.

The controller 210 dispenses from the sample container 104 containing the PRP sample a predetermined amount of the PRP sample into a reaction container 108, and transfers the reaction container 108 onto the heating table 190 to heat the reaction container 108 at a predetermined temperature for a predetermined period of time (Step S32). Note that Step S32 is the same as or similar to Steps S121 and S122 in FIG. 8 The controller 210 dispenses a diluted reagent solution containing an inducer into the reaction container 108 containing the PRP sample, to prepare a measurement specimen containing the PRP sample (Step S33). Note that when multiple measurement items are registered for a single PRP sample, a number of measurement specimens corresponding to the number of the registered measurement items are prepared.

The controller 210 measures the initial absorbance of the specimen containing the PRP sample (Step S34). Note that the absorbance of the specimen is measured while the stir bar in the reaction container 108 is rotated to stir the specimen, however, the initial absorbance for evaluating specimen adequacy may be measured without stirring the specimen within a predetermined time after the addition of the inducer-containing reagent. Next, the controller 213 performs the specimen adequacy evaluation of the PRP sample based on the measurement data from Step S34 (Step S35). When the initial absorbance or the platelet count falls below the lower limit reference value or exceeds the upper limit reference value, the controller 213 outputs error information indicating that the PRP sample used is not adequate as a specimen.

When the evaluation information is obtained indicating that the PRP sample is not adequate as a specimen, the controller 213 displays on the display 4 information for confirming the operator's intention to continue the measurement, and determines whether to continue the measurement (Steps S36, S37). The controllers 210 and 213 may automatically stop the measurement when the initial absorbance or the platelet count of the PRP sample falls below the lower reference value or exceeds the upper reference value (No in Step S37), but preferably provide information to the operator to confirm his or her intention to continue the measurement.

When the evaluation result of the specimen adequacy in Step S35 is positive, i.e., when the initial absorbance or the platelet count of the PRP sample is within the range between the upper and lower limit reference values, it is preferable that the controllers 210 and 213 automatically continue the measurement (Yes in Step S37). In this case, from the viewpoint of improving usability, it is preferable to omit the confirmation with the operator in Step S36.

In an embodiment, when the evaluation result of the specimen adequacy is negative, i.e., when the initial absorbance or the platelet count of the PRP sample is below the lower reference value or exceeds the upper reference value, information for confirming the intention to continue the measurement is displayed on the display 4. For example, along with the error information indicating that the PRP sample is not adequate as a specimen, text information (message) such as "Will the measurement be continued?" or an error code indicating the same is displayed along with a button to select whether to continue the measurement. For example, when an operation signal to continue the measurement is received, the controllers 210 and 213 continue the absorbance measurement of the measurement specimen containing the PRP sample (Yes in Step S37, Step S38), and when no operation signal to continue the measurement is received, the controllers 210 and 213 stop the measurement (No in Step S37).

The controller 210 performs the absorbance measurement of the specimen containing the PRP sample in Step S38, and then performs the absorbance measurement of the specimen containing the PPP sample (Step S39). Note that in the case where the measurement specimen is not being stirred in Step S34, stirring of the specimen is started and the absorbance measurement is performed in Step S38. When the absorbance measurement of the PPP sample is completed, the controller 213 performs analysis of the platelet aggregation function (Step S40), and displays on the display 4 the platelet aggregation function measurement result together with the evaluation result of the specimen adequacy (Step S41). When the controller 213 stops the measurement in Step S37, the controller 213 displays on the display 4 only the evaluation result of the specimen adequacy in Step S41.

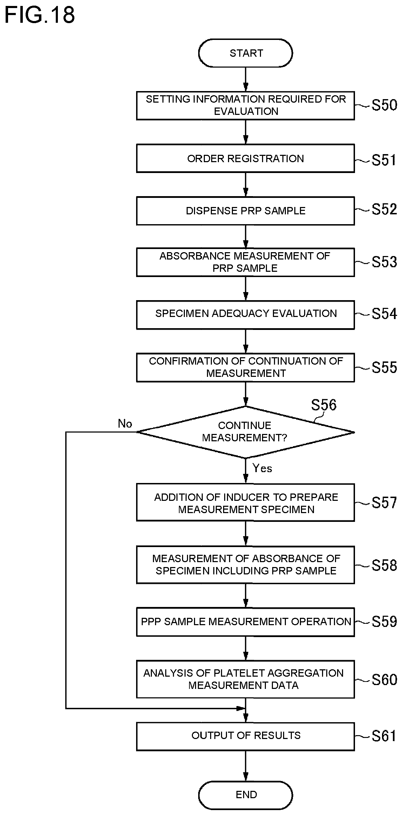

FIG. 18 is a flow diagram illustrating another example of a sample measurement method including specimen adequacy evaluation. An example illustrated in FIG. 18 is common to the example illustrated in FIG. 17 in that, when evaluation information is obtained indicating that a PRP sample is not adequate as a specimen, information is output to confirm the operator's intention to continue measurement. To the contrary, the example illustrated in FIG. 18 differs from the example illustrated in FIG. 17 in that absorbance measurement is performed to evaluate specimen adequacy before adding an inducer-containing reagent to the PRP sample. In the example illustrated in FIG. 18, since a measurement specimen containing the inducer has not been prepared at the time the specimen adequacy evaluation information is obtained, the controllers 210 and 213 can determine whether to prepare the measurement specimen based on the specimen adequacy evaluation information.

As illustrated in FIG. 18, the controller 210 sets information required for evaluating the adequacy of a measurement specimen containing a PRP sample (Step S50). When the measurement start button 30 is operated and a sample rack 105 is thus transported inside the apparatus, the controller 210 controls the sample information reader 103 to read barcodes of sample containers 104 in the sample rack 105 to register a measurement order (Step S51). Then, the controller 210 dispenses, from the sample container 104 that contains a PRP sample, a predetermined amount of the PRP sample into a reaction container 108 (Step S52). Steps S50 to S52 are the same as or similar to Steps S30 to S32 in FIG. 17. However, the reaction container 108 may not be heated in Step S52.

The controller 210 measures the initial absorbance of the PRP sample (Step S53: acquiring third optical information), and performs specimen adequacy evaluation of the PRP sample based on the measurement result (Step S54). When the initial absorbance or the platelet count falls below the lower limit reference value or exceeds the upper limit reference value, the controller 213 outputs error information indicating that the PRP sample used is not adequate as a specimen.

When the evaluation information is obtained indicating that the PRP sample is not adequate as a specimen, the controller 213 displays on the display 4 information for confirming the intention to continue the measurement, and determines whether to continue the measurement (Steps S55, S56). When the initial absorbance or the platelet count of the PRP sample falls below the lower limit reference value or exceeds the upper limit reference value, the controller 213 interrupts the series of measurements and confirms the intention to continue the measurement. The controller 213 may automatically stop the measurement (No in Step S56), but preferably provides the operator with information to confirm his or her intention to continue the measurement.

When the evaluation result of the specimen adequacy is negative, for example, the error information indicating that the PRP sample is not adequate as a specimen is displayed and text information such as "Will a measurement specimen be prepared to continue the measurement?" or an error code indicating the same is also displayed along with a button to select whether to continue the measurement. When receiving an operation signal to continue the measurement, the controllers 210 and 213 continue the measurement of platelet aggregation function (Yes in Step S56). When not receiving an operation signal to continue the measurement, the controllers 210 and 213 stop the measurement (No in Step S56).

When the measurement is continued in Step S56, the controller 210 prepares a measurement specimen containing the PRP sample in Step S57, and measures the absorbance of the measurement specimen (Step S58: acquiring second optical information). Thereafter, the absorbance of the specimen containing the PPP sample is measured (Step S59: obtaining first optical information). That is, in the method illustrated in FIG. 18, the third optical information for evaluating the specimen adequacy is obtained from the PRP sample before the second optical information is obtained. Note that the second optical information is information measured after the reaction has sufficiently progressed, following the lapse of a predetermined time after the reagent inducing the platelet aggregation is added to the platelet-rich plasma. The third optical information is information measured within the predetermined time after the reagent inducing the platelet aggregation is added to the platelet-rich plasma.

As illustrated in FIG. 18, the reaction container 108 is moved as follows; In the measurement of the initial absorbance of the specimen containing the PRP sample (Step S53), The controller unit 210 controls to move the reaction container 108 to the detector 13 to obtain the third optical information. When the measurement is to be continued (Yes in Step S56), the control unit 210 controls to move the reaction container 108 to the measurement sample preparation unit 12, to heat it at a predetermined temperature for a predetermined period of time (the same as or similar to Step S122 in FIG. 8). the control unit 210 controls to move the reaction container 108 to the diluted reagent solution preparation unit 11, to dispense a predetermined amount of diluted reagent solution into the reaction container 108, and to prepare the measurement specimen containing the PRP sample (the same as or similar to Step S123 in FIG. 8). Thereafter, the control unit 210 controls to move the reaction container 108 to the detector 13 to obtain the second optical information.

When the absorbance measurement of the PPP sample is completed, the controller 213 performs analysis of the platelet aggregation function (Step S60), and displays on the display 4 the platelet aggregation function measurement result together with the evaluation result of the specimen adequacy (Step S61). When the controller 213 stops the measurement in Step S56, the controller 213 displays only the evaluation result of the specimen adequacy on the display 4 in Step S61.

FIGS. 19 and 20 illustrates result display screens 900 and 910, which are examples of measurement result display screens, respectively. The result display screen 900 illustrated in FIG. 19 includes a reaction curve display area 901 that displays a reaction curve (agglutination waveform) in which the absorbance is converted into an aggregation rate. Similarly, the result display screen 910 illustrated in FIG. 20 also includes a reaction curve display area 911 that displays the reaction curve. The result display screen 900 is a screen that displays detailed results of a measurement item selected through a measurement item tab 902, whereas the result display screen 910 is a screen that simultaneously displays the results of multiple measurement items. Either of the result display screens 900 and 910 can be displayed on the display 4, and the screen can be switched based on the operator's selection.

The result display screen 900 further includes a detailed information display area 903 and an error information display area 904. In an example illustrated in FIG. 19, the measurement item "ADP 1.0%" is selected, and the measurement results for that measurement item are displayed. The result display screen 900 displays the reaction curve when ADP is added to the PRP sample, and the detailed information display area 903 displays the value of 300 mOD as the initial absorbance "Abs PRP_s" of the specimen containing the PRP sample, and the error information display area 904 displays an error code. That is, the result display screen 900 displays the information regarding the platelet aggregation function and the evaluation information regarding the specimen adequacy of the PRP sample.

The lower limit reference value for the initial absorbance of the specimen containing the PRP sample is set at, for example, 410 mOD. In this case, the initial absorbance of 300 mOD is below the lower limit, and thus the error information is displayed as the evaluation information of the specimen adequacy, indicating that the PRP sample used is not adequate as a specimen , i.e., is abnormal. In the example illustrated in FIG. 19, the error code displayed in the error information display area 904 corresponds to this error information. In the error information display area 904, characters indicating the content of the error, such as "PLT number low", may be displayed together with the error code. "PLT number low" indicates that the platelet count in the sample is low.

The result display screen 910 further includes a result list display area 912. The reaction curve display area 911 and the result list display area 912 of the result display screen 910 display the results of the plurality of measurement items, making it easy to compare the measurement results. Since the initial absorbance of the specimen containing the PRP sample does not vary significantly depending on the type of inducer when the PRP sample is the same, the values of the initial absorbance displayed in the result list display area 912 of FIG. 20 are the same or similar values that are lower than the lower limit reference value. Although the result display screen 910 displays no error code like the one displayed on the result display screen 900, the result display screen 910 may also display the error information that clearly indicates that the PRP sample used is not adequate as a specimen.

The display 4 may display, as the evaluation information of the specimen adequacy, the information that clearly indicates that the PRP sample used is adequate (normal) as a specimen, together with the initial absorbance or the platelet count value of the PRP sample when the initial absorbance or the platelet count calculated from the regression equation is within the reference value range. Alternatively, when the sample is normal, only the initial absorbance or the platelet count value may be displayed, and only when the sample is abnormal, the error information clearly indicating that the sample is abnormal may be displayed along with the initial absorbance or the platelet count value.

The evaluation information of the specimen adequacy when the initial absorbance or the platelet count of the PRP sample falls outside the reference value range may include information that prompt to measure the platelet count using a blood cell counter, as described above, or may also include information regarding the adequacy of the centrifugation conditions for preparing the PRP sample from the blood sample. Also, both pieces of information may be displayed on the result display screen. For example, text information such as "The platelet count may be outside the recommended range for measurement. Please measure the platelet count using a blood cell counter. Also, the centrifugation conditions for the sample preparation may not be appropriate. Please check the centrifugation conditions," or an error code indicating the same may be displayed.

When the initial absorbance or the platelet count of the PRP sample falls outside the reference range, the information displayed on the result display screen may include information regarding other diseases inferred from the initial absorbance or the platelet count of the measured PRP sample. For example, if the platelet count of the PRP sample is low and below the lower limit reference value, this may be due to Bernard-Soulier Syndrome, so text information such as "The platelet count may be outside the recommended range for measurement. Bernard-Soulier Syndrome is suspected," or a code indicating the same, may be displayed on the result display screen.

As described above, the sample measurement method and the sample measurement apparatus 1 having the above configuration make it possible to easily determine the adequacy of a PRP sample as a specimen.

[Remarks]

The present disclosure includes the following items 1-20.

Item 1: A blood coagulation analyzer, comprising:

a sample dispenser configured to dispense a first sample to a first container and dispense a second sample to a second container, wherein the first sample is a platelet-poor plasma prepared from a blood sample and the second sample is a platelet-rich plasma prepared from the blood sample;
a reagent dispenser configured to dispense a reagent that induces a platelet aggregation to the second container;
a detector configured to measure first optical information of the first sample and second optical information of the second sample;
a controller configured to analyze the first and second optical information to provide an analysis result of platelet aggregation function of the blood sample, wherein
the controller is configured to check whether the second sample contains sufficient amount of platelets to obtain accurate analysis result of the platelet aggregation function.

Item 2: The blood coagulation analyzer according to item 1, wherein

the detector is configured to measure third optical information from the second sample, and
the controller is configured to check, based on the third optical information, whether the second sample contains the sufficient amount of platelets.

Item 3: The blood coagulation analyzer according to item 2, wherein
the third optical information is absorbance or transmittance of the second sample.
Item 4: The blood coagulation analyzer according to any one of items 1-3, wherein

the second container accommodates a stirrer bar, and
the detector is configured to rotate the stirrer bar in the second container while measuring the second optical information.

Item 5: The blood coagulation analyzer according to any one of items 1-4, wherein
the detector is configured to start rotation of the stirrer bar after obtaining the third optical information.
Item 6: The blood coagulation analyzer according to item 2 or 3, wherein
the third optical information is obtainable within 10 seconds after the reagent is dispensed to the second container.
Item 7: The blood coagulation analyzer according to item 2, 3 or 6, wherein
the third optical information is obtainable before adding a reagent that induces platelet aggregation to the second container.
Item 8: The blood coagulation analyzer according to item 2, 3, 6 or 7, wherein
the controller is configured to check whether the second sample contains the sufficient amount of platelets, by comparing the third optical information with a reference value.
Item 9: The blood coagulation analyzer according to any one of items 1-8, further comprising a display, wherein
the controller is configured to cause the display to display the analysis result regarding platelet aggregation function and check result on whether the second sample contains the sufficient amount.
Item 10: The blood coagulation analyzer according to item 9, wherein
the controller is configured, in response to determining that the second sample as not containing the sufficient amount of platelets, to cause the display to display error information.
Item 11: The blood coagulation analyzer according to item 9 or 10, wherein
the controller is configured, in response to determining the second sample as not containing the sufficient amount of platelets, to cause the display to display a prompt to measure a platelet count of the second sample using a blood cell counter.
Item 12: The blood coagulation analyzer according to item 2, 3, 6, 7 or 8, wherein
the controller is configured to estimate a platelet count from the third optical information.
Item 13: The blood coagulation analyzer according to item 12, wherein
The controller is configured to check whether the second sample contains the sufficient amount of platelets, by comparing the platelet count with a reference value.
Item 14: The blood coagulation analyzer according to any one of items 1-13, wherein
the controller is configured to analyze the first and second optical information to generate a curve representing a time course progress of a platelet aggregation.
Item 15: The blood coagulation analyzer according to any one of items 1-14, wherein
the controller is configured to analyze the first and second optical information to obtain an index of platelet aggregation.
Item 16: A sample measurement method to measure a platelet aggregation function of a blood sample using a blood coagulation analyzer, the method comprising:

preparing a first sample which is a platelet-poor plasma prepared from a blood sample and a second sample which is a platelet-rich plasma prepared from the blood sample;

combining a reagent that induces a platelet aggregation with the second sample;

acquiring first optical information of the first sample and second optical information of the second sample combined with the reagent;

analyzing the first and second optical information to provide an analysis result of platelet aggregation function of the blood sample; and

checking whether the second sample contains sufficient amount of platelets to obtain accurate analysis result of the platelet aggregation function.

Item 17: The sample measurement method according to item 16, further comprising:

displaying the information about the platelet aggregation function and displaying check result on whether the second sample contains the sufficient amount of platelets.

Item 18: The sample measurement method according to item 16 or 17, wherein

measuring third optical information which is absorbance or transmittance to check whether the second sample contains the sufficient amount of platelets.

Item 19: The sample measurement method according to item 16, 17 or 18, further comprising:

the third optical information is obtainable within 10 seconds after the reagent is added to the second sample.

Item 20: The sample measurement method according to item 19, further comprising generating, based on the first and second optical information, a curve representing a time course progress of a platelet aggregation.

**Claims**

1. A blood coagulation analyzer, comprising:

   a sample dispenser configured to dispense a first sample to a first container and dispense a second sample to a second container, wherein the first sample is a platelet-poor plasma prepared from a blood sample and the second sample is a platelet-rich plasma prepared from the blood sample;

   a reagent dispenser configured to dispense a reagent that induces a platelet aggregation to the second container;

   a detector configured to measure first optical information of the first sample and second optical information of the second sample;

   a controller configured to analyze the first and second optical information to provide an analysis result of platelet aggregation function of the blood sample, wherein

   the controller is configured to check whether the second sample contains sufficient amount of platelets to obtain accurate analysis result of the platelet aggregation function.

2. The blood coagulation analyzer according to claim 1, wherein

   the detector is configured to measure third optical information from the second sample, and

   the controller is configured to check, based on the third optical information, whether the second sample contains the sufficient amount of platelets.

3. The blood coagulation analyzer according to claim 2, wherein
   the third optical information is absorbance or transmittance of the second sample.

4. The blood coagulation analyzer according to any one of claims 1-3, wherein

   the second container accommodates a stirrer bar, and

   the detector is configured to rotate the stirrer bar in the second container while measuring the second optical information.

5. The blood coagulation analyzer according to any one of claims 1-4, wherein
   the detector is configured to start rotation of the stirrer bar after obtaining the third optical information.

6. The blood coagulation analyzer according to claim 2 or 3, wherein
   the third optical information is obtainable within 10 seconds after the reagent is dispensed to the second container.

7. The blood coagulation analyzer according to claim 2 ,3 or 6, wherein
   the third optical information is obtainable before adding a reagent that induces platelet aggregation to the second container.

8. The blood coagulation analyzer according to claim 2, 3, 6 or 7, wherein
the controller is configured to check whether the second sample contains the sufficient amount of platelets, by comparing the third optical information with a reference value.

9. The blood coagulation analyzer according to any one of claims 1-8, further comprising a display, wherein
the controller is configured to cause the display to display the analysis result regarding platelet aggregation function and check result on whether the second sample contains the sufficient amount.

10. The blood coagulation analyzer according to claim 9, wherein
the controller is configured, in response to determining that the second sample as not containing the sufficient amount of platelets, to cause the display to display error information.

11. The blood coagulation analyzer according to claim 9 or 10, wherein
the controller is configured, in response to determining the second sample as not containing the sufficient amount of platelets, to cause the display to display a prompt to measure a platelet count of the second sample using a blood cell counter.

12. The blood coagulation analyzer according to claim 2, 3, 6, 7 or 8, wherein
the controller is configured to estimate a platelet count from the third optical information.

13. The blood coagulation analyzer according to claim 12, wherein
the controller is configured to check whether the second sample contains the sufficient amount of platelets, by comparing the platelet count with a reference value.

14. The blood coagulation analyzer according to any one of claims 1-13, wherein
the controller is configured to analyze the first and second optical information to generate a curve representing a time course progress of a platelet aggregation.

15. The blood coagulation analyzer according to any one of claims 1-14, wherein
the controller is configured to analyze the first and second optical information to obtain an index of platelet aggregation.

16. A sample measurement method to measure a platelet aggregation function of a blood sample using a blood coagulation analyzer, the method comprising:

preparing a first sample which is a platelet-poor plasma prepared from a blood sample and a second sample which is a platelet-rich plasma prepared from the blood sample;
combining a reagent that induces a platelet aggregation with the second sample;
acquiring first optical information of the first sample and second optical information of the second sample combined with the reagent;
analyzing the first and second optical information to provide an analysis result of platelet aggregation function of the blood sample; and
checking whether the second sample contains sufficient amount of platelets to obtain accurate analysis result of the platelet aggregation function.

FIG.1

FIG.2

FIG.3

FIG.4

FIG.5

| Pos. | SAMPLE ID | | ADP | ADP2 | Ara | Col | Col2 | Epi | Ris | |
|---|---|---|---|---|---|---|---|---|---|---|
| 1 PPP | 1234567abcd | ♋ | ✓ | ✓ | | | | | | |
| 2 PRP | | | | | | | | | | |
| 3 PPP | Plateet00: | ♋ | ✓ | | ✓ | | | | | |
| 4 PRP | | | | | | | | | | |
| 5 PPP | Plateet00: 2018/06/22 17:30 | ♋ | ✓ | | ✓ | | | | | |
| 6 PRP | | | | | | | | | | |

**ORDER REGISTRATION**

PLATELET AGGREGATION FUNCTION ORDER ▼   RANK NUMBER   000001

702   701   700

CLEAR ALL   CLEAR ONE LINE

⏮ ⏪ ◀ ▶ ⏩ ⏭

REGISTER   CANCEL

FIG.6

START

ORDER REGISTRATION OF PLATELET AGGREGATION MEASUREMENT — S10

PPP SAMPLE MEASUREMENT OPERATION — S11

PRP SAMPLE MEASUREMENT OPERATION — S12

ANALYSIS OF MEASUREMENT DATA — S13

OUTPUT OF MEASUREMENT RESULTS — S14

END

## FIG.7

PPP SAMPLE MEASUREMENT
OPERATION

↓

DISPENSE PPP SAMPLE INTO
REACTION CONTAINER — S111

↓

DISPENSE DILUENT INTO REACTION
CONTAINER — S112

↓

OPTICAL MEASUREMENT — S113

↓

RETURN

## FIG.8

PRP SAMPLE MEASUREMENT
OPERATION

↓

DISPENSE PRP SAMPLE INTO
REACTION CONTAINER — S121

↓

HEAT PRP SAMPLE FOR
PREDETERMINED TIME — S122

↓

DISPENSE DILUTED REAGENT INTO
REACTION CONTAINER — S123

↓

STIR MEASUREMENT SPECIMEN IN
REACTION CONTAINER — S124

↓

OPTICAL MEASUREMENT — S125

↓

RETURN

FIG.9

FIG.10

(a)

(b)

FIG.11

(a)

(b)

FIG.12

## FIG.13

High limit: 15*10^4
Low limit: 80*10^4

## FIG.14

High limit: 850
Low limit: 410

FIG.15

```
                    ┌─────────────┐
                    │    START    │
                    └─────────────┘
                           │
                           ▼
        ┌──────────────────────────────────┐
        │  SETTING INFORMATION REQUIRED    │~S20
        │         FOR EVALUATION           │
        └──────────────────────────────────┘
                           │
                           ▼
        ┌──────────────────────────────────┐
        │       ORDER REGISTRATION         │~S21
        └──────────────────────────────────┘
                           │
                           ▼
        ║┌────────────────────────────────┐║
        ║│          PPP SAMPLE            │║~S22
        ║│    MEASUREMENT OPERATION       │║
        ║└────────────────────────────────┘║
                           │
                           ▼
        ║┌────────────────────────────────┐║
        ║│    PRP SAMPLE MEASUREMENT      │║~S23
        ║│          OPERATION             │║
        ║└────────────────────────────────┘║
                           │
                           ▼
        ┌──────────────────────────────────┐
        │  SPECIMEN ADEQUACY EVALUATION    │~S24
        └──────────────────────────────────┘
                           │
                           ▼
        ┌──────────────────────────────────┐
        │       ANALYSIS OF PLATELET       │~S25
        │  AGGREGATION MEASUREMENT DATA    │
        └──────────────────────────────────┘
                           │
                           ▼
        ┌──────────────────────────────────┐
        │        OUTPUT OF RESULTS         │~S26
        └──────────────────────────────────┘
                           │
                           ▼
                    ┌─────────────┐
                    │     END     │
                    └─────────────┘
```

FIG.16

FIG.17

```
                        ┌─────────────┐
                        │    START    │
                        └──────┬──────┘
                               │
              ┌────────────────────────────────┐
              │ SETTING INFORMATION REQUIRED FOR │ ～S30
              │          EVALUATION              │
              └────────────────┬───────────────┘
                               │
              ┌────────────────────────────────┐
              │      ORDER REGISTRATION         │ ～S31
              └────────────────┬───────────────┘
                               │
              ┌────────────────────────────────┐
              │   DISPENSE AND HEAT PRP SAMPLE  │ ～S32
              └────────────────┬───────────────┘
                               │
              ┌────────────────────────────────┐
              │          ADD INDUCER            │ ～S33
              └────────────────┬───────────────┘
                               │
              ┌────────────────────────────────┐
              │   ABSORBANCE MEASUREMENT OF     │ ～S34
              │ SPECIMEN CONTAINING PRP SAMPLE  │
              └────────────────┬───────────────┘
                               │
              ┌────────────────────────────────┐
              │  SPECIMEN ADEQUACY EVALUATION   │ ～S35
              └────────────────┬───────────────┘
                               │
              ┌────────────────────────────────┐
              │  CONFIRMATION OF CONTINUATION OF │ ～S36
              │         MEASUREMENT             │
              └────────────────┬───────────────┘
```

S37

No ◁── CONTINUATION OF MEASUREMENT

Yes

CONTINUATION OF ABSORBANCE MEASUREMENT OF SAMPLE CONTAINING PRP SAMPLE ～S38

PPP SAMPLE MEASUREMENT OPERATION ～S39

ANALYSIS OF PLATELET AGGREGATION MEASUREMENT DATA ～S40

OUTPUT OF RESULTS ～S41

END

FIG.18

```
                    ┌─────────────┐
                    │    START    │
                    └──────┬──────┘
                           ▼
         ┌──────────────────────────────────┐
         │ SETTING INFORMATION REQUIRED FOR │ ～S50
         │           EVALUATION             │
         └────────────────┬─────────────────┘
                          ▼
         ┌──────────────────────────────────┐
         │        ORDER REGISTRATION        │ ～S51
         └────────────────┬─────────────────┘
                          ▼
         ┌──────────────────────────────────┐
         │        DISPENSE PRP SAMPLE       │ ～S52
         └────────────────┬─────────────────┘
                          ▼
         ┌──────────────────────────────────┐
         │     ABSORBANCE MEASUREMENT OF    │ ～S53
         │           PRP SAMPLE             │
         └────────────────┬─────────────────┘
                          ▼
         ┌──────────────────────────────────┐
         │    SPECIMEN ADEQUACY EVALUATION  │ ～S54
         └────────────────┬─────────────────┘
                          ▼
         ┌──────────────────────────────────┐
         │  CONFIRMATION OF CONTINUATION OF │ ～S55
         │          MEASUREMENT             │
         └────────────────┬─────────────────┘
                          ▼              S56
                  ◇───────────────◇
       No         │    CONTINUE    │
  ◄─────────────── │  MEASUREMENT?  │
                  ◇───────┬───────◇
                          │ Yes
                          ▼
         ┌──────────────────────────────────┐
         │  ADDITION OF INDUCER TO PREPARE  │ ～S57
         │      MEASUREMENT SPECIMEN        │
         └────────────────┬─────────────────┘
                          ▼
         ┌──────────────────────────────────┐
         │   MEASUREMENT OF ABSORBANCE OF   │ ～S58
         │  SPECIMEN INCLUDING PRP SAMPLE   │
         └────────────────┬─────────────────┘
                          ▼
         ┌──────────────────────────────────┐
         │ PPP SAMPLE MEASUREMENT OPERATION │ ～S59
         └────────────────┬─────────────────┘
                          ▼
         ┌──────────────────────────────────┐
         │  ANALYSIS OF PLATELET AGGREGATION│ ～S60
         │        MEASUREMENT DATA          │
         └────────────────┬─────────────────┘
                          ▼
         ┌──────────────────────────────────┐
         │         OUTPUT OF RESULTS        │ ～S61
         └────────────────┬─────────────────┘
                          ▼
                    ┌─────────────┐
                    │     END     │
                    └─────────────┘
```

## FIG.19

| ITEM | VALUE | UNIT |
|---|---|---|
| ADP1.0% | **** | % |
|  |  |  |
|  |  |  |
|  |  |  |

ERROR INFORMATION:

| CODE/MESSAGE |
|---|
| ⚠ 7050,0000,0000 |
| PLT number low |

DISPLAY ERROR EXPLANATION

| ** | * |
|---|---|
| Abs PPP [mOD] | 67 |
| Abs PRP_s [mOD] | 300 |
| Abs PRP_e [mOD] | **** |
| Abs PRP_min [mOD] | **** |
| Max [%] | **** |
| Fin [%] | **** |
| Lag Phase [sec] | **** |
| AUC [%*sec] | **** |
| VMax [%/sec] | **** |
| VMax time [sec] | **** |
| VMax angle [°] | **** |
|  |  |
|  |  |
|  |  |

## FIG.20

☐ SMOOTHING

| | ADP1.0% | Col1.0% | Col2.0% | |
|---|---|---|---|---|
| | ☑ | ☑ | ☑ | |
| *** | | | | |
| ****** | **** | **** | **** | |
| MEASUREMENT DATE | **** | **** | **** | |
| MEASUREMENT START TIME | **** | **** | **** | |
| Abs PPP [mOD] | 67 | 67 | 67 | |
| Abs PRP_s [mOD] | 300 | 296 | 297 | |
| Abs PRP_e [mOD] | **** | **** | **** | |
| Abs PRP_min [mOD] | **** | **** | **** | |
| Max [%] | **** | **** | **** | |
| Fin [%] | **** | **** | **** | |
| Lag Phase [sec] | **** | **** | **** | |
| AUC [%*sec] | **** | **** | **** | |
| VMax [%/sec] | **** | **** | **** | |
| VMax time [sec] | **** | **** | **** | |

Europäisches
Patentamt

European
Patent Office

Office européen
des brevets

# EUROPEAN SEARCH REPORT

**Application Number**

EP 25 15 6530

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 135 818 A (KENT FREDERICK M ET AL) 23 January 1979 (1979-01-23) | 1-16 | INV. G01N21/25 |
| Y | * col. 1, lines 32-48; col. 2, line 47 to col. 4, line 48; figure 1 * | 1-16 | G01N21/27 G01N21/82 |
| Y | US 2020/400702 A1 (KURONO HIROSHI [JP] ET AL) 24 December 2020 (2020-12-24) * paragraph [0036] - paragraph [0055]; figure 1 * | 1-16 | |
| X | DYSZKIEWICZ-KORPANTY ANNA M ET AL: "Approach to the Assessment of Platelet Function: Comparison between Optical-based Platelet-rich Plasma and Impedance-based Whole Blood Platelet Aggregation Methods", CLINICAL AND APPLIED THROMBOSIS/HEMOSTASIS, 1 January 2005 (2005-01-01), pages 25-35, XP093290466, Retrieved from the Internet: URL:https://journals.sagepub.com/doi/pdf/10.1177/107602960501100103?download=true#:~:text=The%20platelet%20count%20in%20PRP,aggregation%20may%20not%20be%20performed.> | 1-16 | |
| Y | * page 26, section "Platelet Aggregation in Platelet-Richt Plasma (PRP)"; figures 1-4 * | 1-16 | |

-----

-----

-----

-/--

**TECHNICAL FIELDS SEARCHED (IPC)**

G01N

The present search report has been drawn up for all claims

| Place of search | Date of completion of the search | Examiner |
|---|---|---|
| Munich | 30 June 2025 | Weinberger, Thorsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................................

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 25 15 6530

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LAWRIE A S ET AL: "The automation of routine light transmission platelet aggregation", INTERNATIONAL JOURNAL OF LABORATORY HEMATOLOGY, WILEY-BLACKWELL PUBLISHING LTD, GB; US, vol. 36, no. 4, 14 November 2013 (2013-11-14), pages 431-438, XP072388194, ISSN: 1751-5521, DOI: 10.1111/IJLH.12161 * the whole document * ----- | 1,16 | |
| | | | **TECHNICAL FIELDS SEARCHED (IPC)** |

| The present search report has been drawn up for all claims | | |
|---|---|---|
| Place of search | Date of completion of the search | Examiner |
| Munich | 30 June 2025 | Weinberger, Thorsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...............................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 6530

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2025

| Patent document cited in search report | | Publication date | Patent family member(s) | | Publication date |
|---|---|---|---|---|---|
| US 4135818 | A | 23-01-1979 | NONE | | |
| US 2020400702 | A1 | 24-12-2020 | CN | 112098662 A | 18-12-2020 |
| | | | EP | 3754327 A1 | 23-12-2020 |
| | | | JP | 6768118 B1 | 14-10-2020 |
| | | | JP | 2020204558 A | 24-12-2020 |
| | | | US | 2020400702 A1 | 24-12-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Recommendations for the standardization of light transmission aggregometry: a consensus of the working party from the platelet physiology subcommittee of SSC/ISTH. *Journal of Thrombosis and Haemostasis*, 2013, vol. 11, 1183-1189